(19) Europäisches Patentamt
European Patent Office
Office européen des brevets

(11) Veröffentlichungsnummer: **0 440 207 B1**

(12) **EUROPÄISCHE PATENTSCHRIFT**

(45) Veröffentlichungstag der Patentschrift: **07.06.95**

(51) Int. Cl.[6]: **C12N 15/49**, C12N 15/62, C12N 5/10, C12P 21/02, G01N 33/569

(21) Anmeldenummer: **91101224.3**

(22) Anmeldetag: **30.01.91**

(54) **Expression von HIV1- und 2- Polypeptiden und deren Verwendung.**

(30) Priorität: **30.01.90 DE 4002636**

(43) Veröffentlichungstag der Anmeldung:
**07.08.91 Patentblatt 91/32**

(45) Bekanntmachung des Hinweises auf die Patenterteilung:
**07.06.95 Patentblatt 95/23**

(84) Benannte Vertragsstaaten:
**AT BE CH DE DK ES FR GB GR IT LI LU NL SE**

(56) Entgegenhaltungen:
**EP-A- 0 331 961**
**EP-A- 0 416 673**
**WO-A-88/05440**

**DNA Bd. 7, Nr. 6, 1988, Seiten 407 - 416 N. TANESE ET AL.**

(73) Patentinhaber: **BOEHRINGER MANNHEIM GMBH**
**Sandhofer Strasse 112-132**
**D-68305 Mannheim-Waldhof (DE)**

(72) Erfinder: **Kopetzki, Erhard, Dr.**
**Henlestrasse 2**
**W-8122 Penzberg (DE)**
Erfinder: **Bayer, Hubert, Dr.**
**Am Ölschlag 2**
**W-8120 Weilheim (DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**EP 0 440 207 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Verbesserung der Expression von Polypeptiden aus der gag-, env- und pol-Region von HIV1 und HIV2 in Mikroorganismen als Wirtszellen. Sie betrifft weiterhin ein Verfahren und ein Reagenz zum Nachweis von Anti-HIV-Antikörpern in menschlichen Körperflüssigkeiten.

Eine Infektion mit HIV1 oder HIV2 wird üblicherweise durch das Vorhandensein von Anti-HIV-Antikörpern in menschlichen Körperflüssigkeiten nachgewiesen. Zum Nachweis der Anti-HIV-Antikörper werden HIV-Polypeptide benötigt. Demzufolge ist es für die Durchführung einer großen Anzahl von AIDS-Tests erforderlich, immunogen wirkende HIV-Polypeptide in ausreichenden Mengen herzustellen. Diese Herstellung erfolgt im allgemeinen auf gentechnologische Weise in Bakterien.

So ist aus der DE-OS 37 24 016 die Expression von Antigenen aus HIV1 und HIV2 in E. coli als "Nichtfusionsproteine" (ohne heterologen Fusionsanteil) bekannt. Ein Nachteil dieses Verfahrens ist, daß die Expression dieser HIV-Polypeptide in vielen Fällen nur in sehr geringem Ausmaße stattfindet. Ferner sind HIV-Polypeptide oft aufgrund ihrer Hydrophobizität nur schlecht löslich und damit nur schwer zu reinigen.

Weiterhin ist bekannt, HIV-Polypeptide als Fusionsproteine mit HIV-Anteilen und bakteriellen Polypeptidanteilen herzustellen. Dadurch wird in vielen Fällen eine Verbesserung der Expressionshöhe erzielt. So ist aus der EP-A-0 316 659 die Expression von Fusionsproteinen mit Determinanten aus HIV1 und HIV2, einem Trägerpolypeptidanteil, der insbesondere aus der E. coli Chloramphenicol-Acetyltransferase oder der Dihydrofolatreduktase der Maus stammt, und einem Affinitäts-Polypeptid-Anteil, vorzugsweise einem Peptid mit Histidinresten, bekannt. Die Verwendung derartiger Fusionsproteine zum Nachweis von Anti-HIV-Antikörpern wird jedoch eingeschränkt, da Antikörper gegen den verwendeten Fusionspartner in Human-Normalseren auftreten können, was zu falsch positiven Testergebnissen führt.

Aufgabe der Erfindung war es demgemäß, eine hohe Expression von HIV-Fusionspolypeptiden in Mikroorganismen zu erzielen, ohne daß diese Polypeptide immunogene Determinanten aufweisen, die den HIV-Test stören könnten, indem sie mit Human-Normalseren reagieren.

Ein Gegenstand der Erfindung ist daher ein Fusionsprotein mit mindestens einer antigenen oder/und immunogenen Determinante aus der env-, gag- oder/und pol-Region von HIV1 oder/und HIV2, das als N-terminalen Bestandteil die Tetrapeptidsequenz $NH_2$-Met-Tyr-Tyr-Leu enthält.

Überraschenderweise wurde gefunden, daß die Aminosäuresequenz $NH_2$-Met-Tyr-Tyr-Leu der Lactosepermease (lacY) aus E. coli als N-terminaler Bestandteil die Stabilität von HIV-Fusionsproteinen mit mindestens einer antigenen oder/und immunogenen Determinante von HIV1 oder/und HIV2 erhöht und somit eine deutliche Verbesserung der Expressionshöhe in E. coli als Wirtszellen bewirkt. So wird durch N-terminale Fusion der Tetrapeptidsequenz $NH_2$-Met-Tyr-Tyr-Leu die Expressionshöhe von HIV1 und HIV2 Fusionsproteinen bei Verwendung eines geeigneten Expressionsvektors um den Faktor von ca. 10 erhöht. Im menschlichen Normalserum wird keine erhöhte Reaktivität gegen derartige erfindungsgemäße Fusionsproteine gefunden.

Weiterhin können erfindungsgemäße Fusionsproteine als zusätzlichen Bestandteil einen Cluster von mehreren, vorzugsweise 4 bis 30 geladenen hydrophilen Aminosäuren enthalten. Auf diese Weise kann die Löslichkeit von hydrophoben HIV-Fusionsproteinen wesentlich verbessert und dadurch die Bildung von unlöslichen Protein-Aggregationen weitgehend bzw. vollständig verhindert werden, wodurch die Handhabung der Fusionsproteine hinsichtlich Isolierung und Verwendung beim HIV-Test wesentlich verbessert wurde.

Besonders bevorzugt sind erfindungsgemäße Fusionsproteine, die C-terminal eine Abfolge von 4 bis 30 basischen Aminosäuren (Lys und Arg) enthalten. Dadurch läßt sich der isoelektische Punkt eines Proteins zu sehr basischen Werten verschieben, wodurch die Reinigung durch Ionenaustauschchromatographie erheblich erleichtert wird. Der C-terminale poly(Arg,Lys)-Schwanz ist gegebenenfalls nachträglich auf enzymatischem Weg mit Carboxypeptidase B wieder entfernbar (Brewer und Sassenfeld, G.D. Searle, EPA 0 089 626 A2; Brewer und Sassenfeld, Gene 32 (1984) 321-327; Brewer und Sassenfeld, Trends in Biotechnology 3 (1985) 119-122).

Erfindungsgemäße Fusionsproteine enthalten mindestens eine immunogene Determinante aus der env-, gag- oder/und pol-Region von HIV1 oder/und HIV2. DNA-Sequenzen, die für derartige Determinanten kodieren, sind aus dem proviralen Genom von HIV1 bzw. HIV2 nach üblichen molekularbiologischen Verfahren erhältlich. Derartige DNA-Sequenzen können in einen geeigneten Expressionsvektor hinter eine, für die Tetrapeptidsequenz $NH_2$-Met-Tyr-Tyr-Leu (MYYL) kodierende Sequenz im Leserahmen kodiert werden, so daß man ein rekombinantes Gen erhält, das für ein erfindungsgemäßes Fusionsprotein kodiert. Aus dem HIV1-Genom sind die DNA-Sequenzbereiche A', B' und C' bevorzugt.

Die DNA-Sequenz A' ist ein ca. 960 Bp langes Pvu/BglII-Fragment aus der gag-Region von HIV1 und kodiert für 318 Aminosäuren. A' ist in der in SEQ ID NO: 9 dargestellten DNA-Sequenz A enthalten, die für ein Fusionsprotein mit der Aminosäuresequenz I (SEQ ID NO: 1) kodiert, bestehend aus der N-terminalen Tetrapeptidsequenz MYYL, 3 Aminosäuren des Vektors, 317 Aminosäuren aus der gag-Region von HIV1 und einer C-terminalen Vektorsequenz von 13 Aminosäuren.

Die DNA-Sequenz B' ist ein ca. 710 Bp langes PvuII/BspMI-Fragment aus der pol-Region von HIV1, das für 234 Aminosäuren kodiert. B' ist in in SEQ ID NO: 10 dargestellten DNA-Sequenz B enthalten, die für ein Fusionsprotein mit der Aminosäuresequenz II (SEQ ID NO: 2) kodiert, bestehend aus der N-terminalen Tetrapeptidsequenz MYYL, 3 Aminosäuren des Vektors, 234 Aminosäuren aus der pol-Region von HIV1 und einer C-terminalen Vektorsequenz von 6 Aminosäuren.

Die DNA-Sequenz C' ist ein ca. 310 Bp langes RsaI/HindIII-Fragment aus der env-Region von HIV1, das für 101 Aminosäuren kodiert. Die DNA-Sequenz C' ist in der in SEQ ID NO: 11 dargestellten DNA-Sequenz C enthalten, die für ein Fusionspolypeptid mit der Aminosäuresequenz III (SEQ ID NO: 3) kodiert, bestehend aus der N-terminalen Tetrapeptidsequenz MYYL, 22 Aminosäuren des Vektors, 101 Aminosäuren aus der env-Region von HIV1 und einer C-terminalen Vektorsequenz von 12 Aminosäuren.

Durch die Konstruktion bedingt, befinden sich zwischen der für die Peptidsequenz MYYL kodierende DNA-Sequenz und der HIV-DNA, sowie 3'-seitig des HIV-DNA-Fragments noch Sequenzbereiche aus dem Vektor. Diese Vektorsequenzen sollen vorzugsweise für nicht mehr als ungefähr 50 Aminosäuren kodieren. Es ist wichtig, daß diese Aminosäuresequenzen keine antigenen Determinanten bilden können, die von menschlichen Antikörpern erkannt werden und somit störend beim HIV-Test sind.

Die für immunogene HIV-Determinanten kodierenden DNA-Sequenzen können jedoch auch aus synthetischen Oligonukleotiden hergestellt werden, wie weiter unten im Detail beschrieben ist. Die synthetisch hergestellte DNA-Sequenz D' aus der env-Region von HIV2 (vgl. Beispiel 2) kodiert für 115 Aminosäuren. D' ist in der in SEQ ID NO: 12 dargestellten DNA-Sequenz D enthalten, die für ein Fusionspolypeptid mit der Aminosäuresequenz IV (SEQ ID NO: 4) kodiert, bestehend aus der N-terminalen Tetrapeptidsequenz MYYL, zwei Aminosäuren des Vektors, 115 Aminosäuren aus der env-Region von HIV2 und aus einer C-terminalen Vektorsequenz von 9 Aminosäuren.

Weiterhin betrifft die Erfindung auch die Herstellung und Verwendung von "multifunktionellen HIV-Fusionsproteinen", die neben den N-terminalen Fusionssequenzen gleichzeitig mehrere antigene Determinanten der gag-, pol- und env-Regionen von HIV1 und/oder HIV2 enthalten. Diese haben den Vorteil, daß anstelle von mehreren unterschiedlichen HIV-Polypeptiden bzw. Proteinen nur ein multifunktionelles HIV-Fusionsprotein zu fermentieren, zu reinigen und in einen HIV-Antikörpernachweistest einzusetzen ist, was eine erhebliche Kostenverringerung bedeutet. Besonders bevorzugt enthalten die multifunktionellen Fusionsproteine Determinanten von HIV1 und HIV2, so daß in einem Test gleichzeitig Antikörper gegen HIV1 und HIV2 nachweisbar sind. Multifunktionelle Fusionsproteine sind auch möglicherweise interessant als Immunogene, insbesondere für HIV-Vakzine. Die Aminosäuresequenzen und die dafür kodierenden DNA-Sequenzen von besonders bevorzugten multifunktionellen Fusionsproteinen sind aus den Sequenzprotokollen SEQ ID NO: 5 bis 8 bzw. SEQ ID NO: 13 bis 16 ersichtlich. Die Aminosäuresequenz V zeigt ein HIV-Fusionsprotein mit Determinanten aus der env- (101 A.S.) und der gag-Region (317 A.S.) von HIV1. Dieser Aminosäuresequenz entspricht die DNA-Sequenz E, in der die DNA-Sequenzen C' und A' enthalten sind. Die Aminosäuresequenz VI und die dafür kodierende DNA-Sequenz F (mit den DNA-Sequenzen D' und B') zeigen ein HIV-Fusionsprotein mit Determinanten aus der env-Region (114 A.S.) von HIV2 und der pol-Region (234 A.S.) von HIV1. Die Aminosäuresequenz VII und die dafür kodierende DNA-Sequenz G (mit den DNA-Sequenzen D', B', C' und A') zeigen ein HIV-Fusionsprotein mit Determinanten aus der env-Region (114 A.S.) von HIV2, sowie der pol- (234 A.S.), env- (101 A.S.) und gag-Region (317 A.S.) von HIV1.

Weiterhin ein Gegenstand der Erfindung sind rekombinante DNA-Moleküle, die für erfindungsgemäße Fusionsproteine kodieren. Diese DNA-Sequenzen setzen sich aus der für die Tetrapeptidsequenz $NH_2$-Met-Tyr-Tyr-Leu kodierenden DNA-Sequenz, gegebenenfalls aus der durch die Konstruktion eingeführten Vektorsequenzen, aus den für die HIV-Determinanten kodierenden DNA-Sequenzen und gegebenenfalls aus der für ein Cluster von mehreren geladenen Aminosäuren kodierenden DNA-Sequenz zusammen. Ein Beispiel hierfür ist die Aminosäuresequenz VIII und die dafür kodierende DNA-Sequenz H (mit den DNA-Sequenzen D', B' und C', sowie dem größten Teil der DNA-Sequenz A'), welche ein HIV-Fusionsprotein mit Determinanten aus der env- Region (114 A.S.) von HIV2, der pol-(234 A.S.), env- (101 A.S.) und gag-Region (287 A.S.) von HIV1, sowie eine C-terminale poly(Lys,Arg)-Sequenz (13 A.S.) zeigt.

Die für immunogene HIV-Determinanten kodierenden DNA-Sequenzen sind wie zuvor beschrieben direkt aus der proviralen DNA oder durch chemische Gensynthese erhältlich. Abbildung 1 zeigt die Bereiche von HIV1 bzw. die entsprechende Region von HIV2, aus denen die erfindungsgemäß besonders bevorzugten proviralen DNA-Sequenzen stammen. Zur weiteren Verbesserung der Expression können synthetische DNA-Sequenzen, die für entsprechende HIV-Polypeptide kodieren, unter Berücksichtigung der dem Fachmann bekannten Codonnutzung des verwendeten Wirtsorganismus (z.B. E. coli) hergestellt werden. Beim Design von synthetischen Genen ist weiterhin darauf zu achten, daß die Ausbildung von Sekundärstrukturen minimiert wird, vorzugsweise können auch geeignete singuläre Restriktionsschnittstellen eingeführt werden. Die zur Herstellung der synthetischen DNA-Sequenz D' verwendeten Desoxyoligonukleotide und die Klonierungsstrategie sind im Detail in Abbildung 2, den Sequenzprotokollen 17 - 26 und im Beispiel 2.2 dargestellt.

Weiterhin ein Gegenstand der vorliegenden Erfindung sind rekombinante Vektoren mit mindestens einer Kopie einer oder mehrerer erfindungsgemäßer rekombinanter DNA-Sequenzen. Der Vektor kann sich in das Genom der Wirtszelle integrieren (z.B. Vektoren des Bakteriophagen λ in E.coli), vorzugsweise liegt er jedoch extrachromosomal, besonders bevorzugt in hoher Kopienzahl vor. Vorzugsweise ist der rekombinante Vektor zur Genexpression in Mikroorganismen, insbesondere E. coli geeignet und besitzt die dafür erforderlichen genetischen Elemente (z.B. Origin of Replication, Selektionsmarker, Promotor, Terminator, etc.).

Besonders bevorzugt sind Vektoren, in denen die für ein erfindungsgemäßes Fusionsprotein kodierende DNA-Sequenz sich unter Kontrolle eines regulierbaren Promotors befindet (z.B. trp/lac-Fusionspromotor für die Genexpression in E. coli). Weiterhin bevorzugt ist, wenn der rekombinante Vektor am 3'-Ende des Fusionsgens einen oder mehrere Transkriptionsterminatoren enthält (z.B. die Terminatoren $T_1$ und $T_2$ aus dem E. coli rrnB ribosomalen RNA Operon, Brosius et al., J. Mol. Biol. 148 (1981), 107-127). Ein Beispiel für einen besonders geeigneten Expressionsvektor in E. coli ist pKK233-2 (LKB-Pharmacia) mit dem regulierbaren trc-Promotor und den Terminatoren $T_1$ und $T_2$.

Ein weiterer Gegenstand der Erfindung ist ein Mikroorganismus, der mit einem erfindungsgemäßen rekombinanten Vektor oder einem erfindungsgemäßen rekombinanten DNA-Molekül transformiert ist. Vorzugsweise ist dieser Mikroorganismus eine E. coli-Zelle.

Als E. coli Stämme sind z.B. die Stämme RM82lac$^+$ (eine Methionin und Lactose Revertante von ED8654, Murray et al., Mol. Gen. Genet. 150 (1977), 53-61), HB101 (Maniatis et al. (1982), Molecular Cloning: A Laboratory Manual. Cold Spring Harbor Laboratory Press, Cold Spring Harbor, New York), und K165 (Neidhardt und Van Bogelen, Biochem. Biophys. Res. Commun. 100 (1981) 884-900), aber auch weitere Stämme geeignet.

In einer bevorzugten Ausführungsform der vorliegenden Erfindung ist der transformierte E. coli Stamm mit einem weiteren kompatiblen Plasmid cotransformiert. Bei Verwendung eines durch Lactose regulierbaren Promotors (z.B. lac-Promotor, trp/lac-Fusionspromotor) enthält das kompatible Plasmid z.B. ein lacI-(Fabaugh, Nature 274 (1978) 765-769) oder ein lacI$^q$-Repressorgen (Calos, Nature 274 (1978) 762-765) wie z.B. das Plasmimd pFDX500 ein pACYC177-Derivat (Chang und Cohen, J. Bacteriol. 134 (1978) 1141-1156) oder das F'-Episom aus E. coli JM109 (Yanisch-Perron et al., Gene 33 (1985) 103-109), welche das lacI$^q$-Repressorgen enthalten.

Die zusätzliche Anwesenheit solcher Repressorplasmide kann die Expression von HIV-Fusionsproteinen verbessern. Es kann jedoch auch ein cotransformiertes Repressorplasmid verwendet werden, das zusätzlich ein Gen für die in E. coli seltene t-RNA Arg (Anticodone: AGA, AGG) enthält, z.B. das dnaY-lacI$^q$-Plasmid pUBS500 (EP-A 0 368 342). Überraschenderweise wurde nämlich gefunden, daß die Expression von HIV-Fusionsproteinen durch Anwesenheit eines dnaY-lacI$^q$-Plasmids noch weiter verbessert werden kann.

Weiterhin ein Gegenstand der Erfindung sind Verfahren zur Gewinnung der HIV-Fusionsproteine, bei denen man geeignete Mikroorganismen, vorzugsweise E. coli-Zellen mit einem erfindungsgemäßen rekombinanten DNA-Molekül oder Vektor transformiert, auf dem sich ein für ein erfindungsgemäßes HIV-Fusionsprotein kodierendes Gen befindet, und das Fusionsprotein nach üblichen biochemischen Verfahren aus den Zellen oder dem Medium gewinnt.

Dabei können erfindungsgemäße Fusionsproteine, die eine Trennhilfe enthalten, z.B. ein Cluster von geladenen Aminosäuren, leicht durch Ionenaustausch-Chromatographie von den übrigen zellulären Bestandteilen abgetrennt werden.

In einem bevorzugten Verfahren zur Gewinnung der erfindungsgemäßen HIV-Fusionsproteine wird die Expression des für das Fusionsprotein kodierenden Gens während der Anzuchtphase reprimiert, z.B. durch Anwesenheit eines Repressors. Eine Genexpression findet dann erst bei Induzierung, z.B. durch Zugabe eines Induktors statt.

Bevorzugt ist weiterhin ein Verfahren, bei dem man einen Mikroorganismus, in dem das rekombinante Fusionsprotein kodierende Gen und ein Repressor-Gen gleichzeitig (z.B. auf einem Plasmid oder auf zwei kompatiblen Plasmiden) vorliegen, verwendet. So kann man die HIV-Fusionsproteine aus Zellen gewinnen, die mit einem Repressor-Plasmid, z.B. dem lacI$^q$-Repressorplasmid pFDX500 cotransformiert sind. Auf diese Weise kann eine bessere Reprimierung der Genexpression während der Anzuchtphase erreicht werden.

Bevorzugt ist auch ein Verfahren, bei dem der Mikroorganismus das erfindungsgemäße rekombinante Gen, ein Repressor-Gen und zusätzlich das E. coli dnaY-Gen ($tRNA^{Arg}$) enthält. Dafür kann man z.B. Zellen verwenden, die anstelle von pFDX500 mit dem dnaY-$lacI^{q}$-Plasmid pUBS500 cotransformiert sind. Die Anwesenheit des dnaY-Gens in einem transformierten Mikroorganismus kann zu einer weiteren Erhöhung der Expression von erfindungsgemäßen Fusionsproteinen führen.

Weiterhin ein Gegenstand der Erfindung ist ein Reagenz zur Bestimmung von Anti-HIV-Antikörpern.

Dieses Reagenz enthält

(a) eines oder mehrere erfindungsgemäße Fusionsproteine mit mindestens einer antigenen oder/und immunogenen Determinante von HIV1 oder/und HIV2, die entweder an eine Festphase gebunden sind oder so modifiziert sind, daß sie während eines Inkubationsschritts leicht an eine geeignete spezielle Festphase leicht binden,

(b) einen Inkubationspuffer, der die physiologische Bindung von Anti-HIV-Antikörpern an die antigenen Determinanten der Fusionsproteine erlaubt und gleichzeitig unspezifische Bindungen unterdrückt,

(c) ein Nachweisreagenz zur Erkennung der Antigen-gebundenen Antikörper und

(d) ein System zur Quantifizierung der gebundenen spezifischen Antikörper.

Das Antigen kann z.B. als Antigen-Hapten-Konjugat vorliegen, während auf der Festphase Moleküle immobilisiert sind, die mit dem Hapten reagieren. Vorzugsweise sind erfindungsgemäße Antigene mit Biotin konjugiert und binden daher mit hoher Affinität an ein mit Streptavidin beschichtetes Reaktionsgefäß. Es ist jedoch ebenfalls möglich, die Antigene direkt nach anderen üblicherweise bekannten Verfahren adsorptiv an eine feste Matrix zu binden (vergleiche Beispiel 6). Als Inkubationspuffer sind alle üblicherweise für immunchemische Reaktionen verwendeten Puffer geeignet. Vorzugsweise verwendet man PBS (0,15 mol/l Na-Phosphat, 0,9 % NaCl, pH 7,2) mit 10 % Kälberserum und 0,05 % Tween 20.

Als Nachweisreagenz zur Erkennung der Antigen-gebundenen Antikörper sind Konjugate aus einem Enzym (z.B. Peroxidase) und polyklonalen Antikörpern gegen den Fcγ-Teil von Human-IgG oder auch enzymkonjugierte HIV-Antigene geeignet.

Durch Zugabe eines Substrats, das von dem mit Anti-Fcγ-Antikörper bzw. HIV-Antigen konjugierten Enzym gespalten wird und dessen Umsetzung meßbar ist, kann man die gebundenen spezifischen Anti-HIV-Antikörper quantitativ bestimmen. Verwendet man Peroxidase als Enzym, so ist als Substrat z.B. ABTS® (2,2'-Azino-di-[3-ethylbenzthiazolin-sulfonsäure(6)]-diammoniumsalz) geeignet.

Die folgenden Beispiele sollen die Erfindung in Verbindung mit den Sequenzprotokollen 1 - 26 und den Abbildungen 1 und 2 verdeutlichen (dabei bedeuten AS: Aminosäure, B: Base). Es zeigen:

SEQ ID NO:1 — die Aminosäure- (I) und SEQ ID NO: 9 die DNA-Sequenz (A) eines HIV-Fusionsproteins mit einer Determinante (AS 8 bis AS 324, B 20 bis B 972; dabei bedeutet AS: Aminosäure, B: Base) aus der gag-Region von HIV1 (HIV1gag-p17-p24-p15);

SEQ ID NO:2 — die Aminosäure- (II) und SEQ ID NO: 10 die DNA-Sequenz (B) eines HIV-Fusionsproteins mit einer Determinante (AS 8 bis 241, B 21 bis 725) aus der pol-Region von HIV1 (HIV1pol-p32);

SEQ ID NO:3 — die Aminosäure- (III) und SEQ ID NO: 11 die DNA-Sequenz (C) eines HIV-Fusionsproteins mit einer Determinante (AS 27 bis 127, B 79 bis 383) aus der env-Region von HIV1 (HIV1env-gp41);

SEQ ID NO:4 — die Aminosäure- (IV) und SEQ ID NO: 12 die DNA-Sequenz (D) eines HIV-Fusionsproteins mit einer Determinante (AS 7 bis 121, B 19 bis 363) aus der env-Region von HIV2 (HIV2env-gp32);

SEQ ID NO:5 — die Aminosäure- (V) und SEQ ID NO: 13 die DNA-Sequenz (E) eines HIV-Fusionsproteins mit Determinanten (AS 27 bis 44 5, B 79 bis 133 5) aus der env- und gag-Region von HIV1 (HIV1(env-gp41)-(gag-p17-p24-p15));

SEQ ID NO:6 — die Aminosäure- (VI) und SEQ ID NO: 14 die DNA-Sequenz (F) eines HIV-Fusionsproteins mit Determinanten (AS 7 bis 359, B 19 bis 1079) aus der env-Region von HIV2 und der pol-Region von HIV1 (HIV2(env-gp32)-HIV1(pol-p32));

SEQ ID NO:7 — die Aminosäure- (VII) und SEQ ID NO: 15 die DNA-Sequenz (G) eines HIV-Fusionsproteins mit Determinanten (AS 7 bis 786, B 19 bis 2358) aus der env-Region von HIV2, der pol-, env- und gag-Region von HIV1 (HIV2(env-gp32)-HIV1-(pol-p32)-(env-gp41)-(gag-p17-p24-p15));

SEQ ID NO: 8 — die Aminosäure- (VIII) und SEQ ID NO: 16 die DNA-Sequenz (H) eines HIV-Fusionsproteins mit Determinanten (AS 7 bis 756, B 19 bis 2268) aus der env-Region von HIV2, der pol-, env- und gag-Region von HIV1 und einer C-terminalen poly(Lys, Arg)-Sequenz (AS 758 bis Ende, B 2272 bis Ende) (HIV2(env-gp32)-HIV1(pol-p32)-(env-gp41)-(gag-p17-p24-p15)-poly(Lys,Arg));

| | |
|---|---|
| SEQ ID NO:17-26 | die Nukleotidsequenzen der zur Herstellung der synthetischen HIV2(env-gp32)-DNA-Sequenz (D') verwendeten Desoxyoligonukleotide; |
| Abb. 1 | die Lokalisierung der HIV-Determinanten auf dem HIV1 Genom; |
| Abb. 2 | die Klonierungsstrategie zur Herstellung der HIV2(env-gp32)-DNA-Sequenz (D'). |

Beispiele:

**Beispiel 1**

1. HIV-Antigene

Zur Manipulation von DNA wurden Standardmethoden benutzt, wie sie bei Maniatis et al. (1982) in Molecular Cloning: A Laboratory Manual, Cold Spring Harbor Laboratory, Cold Spring Habor, New York und Sherman et al. (1986) in Methods in Yeast Genetics, Cold Spring Harbor Laboratory, Cold Spring Habor, New York, beschrieben sind.

1.1 Charakterisierung der verwendeten HIV1-DNA-Fragmente

Die verwendete HIV1 Provirus DNA stammt aus dem HIV1-λ-Phagen Klon λWF1.13 aus dem Labor von B.H. Hahn (The University of Alabama at Birmingham, Birmingham, USA). Der λWF1.13 Klon enthält die nicht permutierte fast vollständige Provirus DNA (ca. 9. kBp langes SstI/SstI-Restriktionsendonukleasefragment). Die provirale DNA stammt aus einem Virusisolat aus peripheren Blutzellen eines an AIDS verstorbenen Patienten. Teilbereiche der λWF1.13-DNA (ca. 9 kBp langes SstI/SstI-, ca. 5.3 kBp langes SstI/EcoRI- und ca. 3.7 kBp langes EcoRI/SstI-Fragment) wurden im Labor von Prof. Dr. Dr. H. Wolf (Pettenkofer Institut, München) in das ca. 2.7 kBp lange SstI-bzw. SstI/EcoRI-Vektorfragment des E. coli Vektors pUC18 (Yanisch-Perron et al., Gene 33 (1985) 102-119, Vieira und Messing, Gene 19 (1982) 259-268) subkloniert. Die Konstruktionen wurden mit pUC18__WF113__SstI/SstI-9, pUC18__WF113__SstI/EcoRI-19 und pUC18__WF113__EcoRI/SstI-28 bezeichnet. Die DNA-Sequenzen der zur Expression von HIV1-Fusionsproteinen verwendeten retroviralen DNA Teilbereiche der gag- (ca. 960 Bp langes PvuII/BglII-Fragment) pol- (ca. 710 Bp langes PvuII/BspMI-Fragment) und env-Region (ca. 310 Bp langes RsaI/HindIII-Fragment) wurden nach der Didesoxykettenabbruch-Reaktion nach Sanger (Sanger et al., J. Mol. Biol. 143 (1980) 161-178) sequenziert. Die Lokalisierung dieser DNA-Fragmente auf dem HIV-Genom ist aus Abb. 1 ersichtlich. Das Fragment A' aus der gag-Region ist in der DNA-Sequenz A enthalten und kodiert für die HIV1 immunogene Determinante des Fusionsproteins I. Das Fragment B' aus der pol-Region ist in der DNA-Sequenz B enthalten und kodiert für die HIV1 immunogene Determinante des Fusionsproteins II. Das Fragment C' aus der env-Region ist in der DNA-Sequenz C enthalten und kodiert für die HIV1 immunogene Determinante des Fusionsproteins III.

1.2 Subklonierung der verwendeten HIV1-gp41 DNA aus der env-Region des Virusisolats WF1.13 (DNA-Sequenz C')

Aus dem Plasmid pUC18__WF113__EcoRI/SstI-28 wurde ein ca. 310 Bp langes RsaI/HindIII-Fragment der "envelope" Region isoliert und in das 2.7 kBp lange pUC18 HincII/HindIII-Vektorfragment ligiert (Konstruktion: pUC18__WF113__RsaI/HindIII). Die subklonierte DNA-Sequenz des RsaI/HindIII- Fragments des Virusisolates WF1.13 entspricht der DNA-Sequenz des Virusisolates WMJ-1 von Position 1638 bis Position 1943 (Starcich et al., Cell 45 (1986) 637-648). Aus dem Plasmid pUC18__WF113__RsaI/HindIII wurde ein ca. 310 langes BamHI/HindIII-Fragment isoliert und anschließend in das 5.2 kBp lange mit BamHI und HindIII geschnittene Vektorfragment von pUR288 (Rüther und Müller-Hill, EMBO Journal 2 (1983) 1791-1794) ligiert (Konstruktion:
pUR288__WF113__BamHI/HindIII). Die durch Umkonstruktion entstandenen DNA Übergänge wurden sequenziert.

1.3 Subklonierung der verwendeten HIV1-p17-p24-p15 DNA aus der gag-Region des Virusisolats WF1.13 (DNA-Sequenz A')

Das Plasmid pUC18__WF113__SstI/EcoRI-19 wurde mit PvuII verdaut, die DNA-Enden mit einem EcoRI-Linker (5'-GGAATTCC-3') versehen, die Plasmid-DNA mit BglII nachgeschnitten und die überhängenden 5'-Enden der BglII-Schnittstelle mit Klenow Polymerase aufgefüllt. Danach wurde mit EcoRI nachgeschnitten, das ca. 960 Bp lange EcoRI/BglII (blunt)-Fragment isoliert und in das ca. 4,6 KBp lange EcoRI/HindIII (blunt) pKK233-2/MYYL Vektorfragment (siehe Beispiel 2.4) ligiert (Konstruktion: pKK233-2/MYYL-p17-p24-p15). Zur Herstellung des EcoRI/HindIII (blunt) pKK233-2/MYYL Vektorfragments wurde das Plasmid pKK233-2/MYYL mit HindIII verdaut, die überhängenden 5'-Enden der HindIII-Schnittstelle mit Klenow Polymerase aufgefüllt, die Plasmid-DNA mit EcoRI nachgespalten und danach das Vektorfragment isoliert.

1.4 Subklonierung der verwendeten HIV1pol-p32 DNA aus der pol-Region des Virusisolats WF1.13 (DNA-Sequenz B')

Aus dem Plasmid pUC18__WF113__SstI/EcoRI-19 wurde ein ca. 970 Bp langes Asp718I/NdeI-Fragment aus der "pol"-Region von HIV1, welches das verwendete ca. 710 Bp lange PvuII/BspMI-Fragment enthält, isoliert, die überhängenden 5'-Enden mit Klenow Polymerase aufgefüllt und in die HincII-Schnittstelle des pUC18 Vektors (Yanisch-Perron et al. (1985) supra) ligiert (Konstruktion:
pUC18__WF113__INT).

Das Plasmid pUC18__WF113__INT wurde mit PvuII verdaut, die DNA-Enden mit einem EcoRI-Linker (5'-CGGAATTCCG-3') versehen, die Plasmid-DNA mit BspMI verdaut und die überhängenden 5'-Enden der BspMI-Schnittstelle mit Klenow-Polymerase aufgefüllt. Danach wurde mit EcoRI nachgespalten, das ca. 720 Bp lange EcoRI/BspMI (blunt)-Fragment isoliert und in das 4.6 KBp lange EcoRI/HindIII (blunt)-pKK233-2/MYYL Vektorfragment ligiert (Konstruktion: pKK233-2/MYYL-pol-p32). Zur Herstellung des EcoRI/HindIII (blunt) pKK233-2/MYYL Vektorfragments wurde das Plasmid pKK233-2/MYYL (siehe Beispiel 2.4) mit HindIII verdaut, die überhängenden 5'-Enden der HindIII-Schnittstelle mit S1-Nuclease entfernt, die DNA mit EcoRI nachgeschnitten und danach das Vektorfragment isoliert.

**Beispiel 2**

HIV2 Antigen

2.1 Auswahl des antigenen Bereiches und Gendesign

Anhand der aus der Arbeitsgruppe von L. Montagnier publizierten HIV2 DNA Sequenz und davon abgeleiteten Proteinsequenzen (Guyader et al., Nature 326 (1987) 662-668) wurde aus dem Transmembranbereich des HIV2 "envelope" Proteins gp32 nach Antigenindexanalyse (Jameson und Wolf, CABIOS 4 (1988) 181-186; Wolf et al., CABIOS 4 (1988) 187-191) der HIV2 gp32 Bereich von Aminosäure 48-162 ausgewählt. Das synthetische HIV2-gp32 Teilgen wurde hinsichtlich "Codonusage" an die bevorzugt benutzten Codone der für die Expression des HIV2-gp32 Teilproteins vorgesehenen Wirtsorganismen E. coli und Hefe angepaßt. Beim Gendesign wurde die Ausbildung von Sekundärstrukturen minimiert, und zusätzlich wurden im Hinblick auf Fusionsproteine geeignete singuläre Restriktionsendonukleaseschnittstellen eingeführt.

2.2 Gensynthese

Die zur Herstellung der synthetischen DNA-Sequenz D' verwendeten Desoxyoligonukleotide und die Klonierungsstrategie sind im Detail in den Sequenzprotokollen SEQ ID NO: 17 bis 26 und in Abb. 2 dargestellt.

Prinzip:

Jeweils 5 synthetische Desoxyoligonukleotide wurden zu 2 ca. 200 Bp langen Genblöcken mit jeweils geeigneten Restriktionsschnittstellen an ihren Enden zusammengefügt, gelelektrophoretisch gereinigt, mit Restriktionsenzymen geschnitten und in die Polylinkerregion des E. coli pUC18 Vektors subkloniert. Mittels DNA-Sequenzierung wurde die vorgegebene DNA-Sequenz der beiden subklonierten Genblöcke bestätigt. Danach wurden die beiden DNA Fragmente über die singuläre KpnI Schnittstelle im pUC18 Vektor verknüpft (Konstruktion: pUC18__HIV2-gp32).

2.3 Konstruktion des Plasmids pUC18__HIV2-gp32

A) Die Desoxyoligonukleotide 1 (SEQ ID NO: 17), 2 (SEQ ID NO: 18), 10 (SEQ ID NO: 26), 9 (SEQ ID NO: 25) und 8 (SEQ ID NO: 24) (siehe Abb. 2) wurden in einem Reaktionsansatz (Reaktionspuffer: 12,5 mol/l Tris/HCl, pH 7,0 und 12,5 mol/l $MgCl_2$) annealt, das Hybridisierungsprodukt mit BamHI und KpnI verdaut, das ca. 200 Bp lange BamHI/KpnI-Fragment isoliert und in das mit BamHI und KpnI verdaute pUC18-Vektorfragment subkloniert (Konstruktion: pUC18__HIV2/BK).

B) Die Desoxyoligonukleotide 3 (SEQ ID NO: 19), 4 (SEQ ID NO: 20), 7 (SEQ ID NO: 23), 6 (SEQ ID NO: 22) und 5 (SEQ ID NO: 21) (siehe Abb. 2) wurden wie unter Punkt A) beschrieben annealt, das Hybridisierungsprodukt mit KpnI und HindIII verdaut, das ca. 200 Bp lange KpnI/HindIII-Fragment isoliert und in das mit KpnI und HindIII verdaute pUC18-Vektorfragment subkloniert (Konstruktion: pUC18__HIV2/KH).

C) Aus dem Plasmid pUC18__HIV2/KH wurde das ca. 200 Bp lange KpnI/HindIII-Fragment isoliert und in das ca. 2.7 KBp lange KpnI/HindIII pUC18__HIV2/BK-Vektorfragment ligiert (Konstruktion: pUC18__HIV2-gp32).

In der Konstruktion pUC18__HIV2-gp32 steht die HIV2-gp32 DNA unter lac-Promotor/Operator Kontrolle. Die HIV2-gp32 DNA bildet einen offenen Leserahmen mit dem lacZ-αPeptid des pUC18 Polylinkers, so daß bei der Expression in E. coli ein lacZ(αPeptid)-HIV2-gp32 Fusionsprotein synthetisiert wird. Der N-terminale lacZ(αPeptid)-Anteil ist 13 Aminosäuren lang.

2.4 Konstruktion des Basisexpressionsplasmids pKK233-2/MYYL

Der E. coli ATG-Expressionsvektor pKK233-2 (trc-Promotor, NcoI-PstI-HindIII Polylinker, rrnB-Fragment mit dem 5S-rRNA Gen und den Transkriptionsterminatoren T1 und T2) stammt von der Firma LKB-Pharmacia (Uppsala). Die singuläre EcoRI-Restriktionsschnittstelle in pKK233-2 wurde durch Restriktionsspaltung mit EcoRI, Auffüllung der überhängenden 5'-Enden mittels Klenow Polymerase und anschließende "blunt-end" Ligierung zerstört (Konstruktion: pKK233-2/E). Der NcoI-PstI-HindIII Polylinker im Plasmid pKK233-2/E wurde gegen einen NcoI-EcoRI-XbaI-HindIII Polylinker ausgetauscht (Konstruktion: pKK233-2/MYYL). Dieser ermöglicht die Konstruktion von Fusionsproteinen, die N-terminal mit den 4 N-terminalen Aminosäuren der Lactosepermease (lacY) beginnen.

Zur Herstellung des Plasmids pKK233-2/MYYL wurde das Plasmid pKK233-2/E mit NcoI und HindIII verdaut, das ca. 4.6 kBp lange pKK233-2/E NcoI/EcoRI-Vektorfragment isoliert und mit dem NcoI-EcoRI-XbaI-HindIII Polylinker ligiert. Der NcoI-EcoRI-XbaI-HindIII-Polylinker wurde mittels Hybridisierung aus den folgenden zwei Desoxyoligonukleotiden hergestellt.

```
                         XbaI
( NcoI)          EcoRI    HindIII
CATGTACTATTTAGAATTCTAGA
    ATGATAAATCTTAAGATCTTCGA
    MetTyrTyrLeu ------>
```

2.5 Konstruktion des Plasmids pKK233-2/MYYL__HIV2-gp32

In das ca. 4.6 kBp lange pKK233-2/MYYL EcoRI/HindIII-Vektorfragment wurde das ca. 400 Bp lange EcoRI/HindIII-Fragment aus pUC18__HIV2-gp32 ligiert. Das lacY-HIV2 Fusionsprotein IV ist in SEQ ID NO: 4 und die codierende DNA Sequenz D ist in SEQ ID NO: 12 dargestellt.

2.6 Expression von HIV2-gp32 Fusionsproteinen in E. coli

Als Wirtsstämme wurden die bekannten E. coli K12 Stämme RM82lac$^+$ (met$^+$, lac$^+$ Revertante von ED8654; Murray et al., Mol. Gen. Genet. 150 (1977) 53-61), HB101 (Maniatis et al., (1982) supra und K165 (Neidhardt und Van Bogelen, Biochem. Biophys. Res. Commun. 100 (1981) 884-900) benutzt. Die Expression von HIV2-gp32 Fusionsproteinen wurde in Abwesenheit und Anwesenheit des lacI$^q$-Repressorplasmids pFDX500 (supra, siehe Seite 8) untersucht. RM82lac$^+$, HB101 und K165 bzw. RM82lac$^+$, HB101 und K165,

cotransformiert mit dem $lacI^q$-Repressorplasmid pFDX500, wurden jeweils mit den HIV2-gp32 Expressionsplasmiden pUC18_HIV2-gp32 und pKK233-2/MYYL_HIV2-gp32 transformiert und anschließend bei 30 °C in DYT-Medium (16 g Bactotrypton, 10 g Hefeextrakt, 5 g NaCl pro Liter), supplementiert mit 50 mg/l Ampicillin bzw. 50 mg/l Ampicillin plus 50 mg/l Kanamycin, angezogen. Nach Erreichen einer optischen Dichte von 0.6 bis 0.8 bei 550 nm wurden die Zellen mit IPTG (Isopropyl-β-D-thiogalactopyranosid, Endkonzentration 1 mmol/l) induziert. Nach einer Induktionszeit von 5 und 20 Stunden wurden 1 ml Proben entnommen, die Zellen abzentrifugiert, mit 10 mmol/l Phosphatpuffer, pH 6.8, gewaschen und bis zur Weiterverarbeitung bei -20 ° gelagert.

2.7 Zellaufschluß, SDS-Polyacrylamid Gelelektrophorese (PAGE) und Westernblot-Analyse

Das Zellpellet wurde in 0.25 ml 10 mmol/l Phosphatpuffer, pH 6.8, und 1 mmol/l EDTA resuspendiert, und die Zellen durch Ultraschallbehandlung aufgeschlossen. Nach Zentrifugation wurde der Überstand mit 1/5 Volumen 5xSDS-Probenpuffer (1xSDS-Probenpuffer: 50 mmol/l Tris/HCl, pH 6.8, 1% SDS, 1% Mercaptoethanol, 10% Glycerin, 0.001% Bromphenolblau) versetzt, die unlösliche Zelltrümmerfraktion in 0.3 ml 1xSDS-Probenpuffer und 6 mol/l Harnstoff resuspendiert, die Proben 5 Minuten bei 95 °C inkubiert, zentrifugiert, die Proteine durch SDS-Polyacrylamid Gelelektrophorese aufgetrennt (Laemmli, Nature 227 (1970) 680-685) und anschließend mit Coomassie Brilliant Blue R angefärbt. Alternativ bzw. parallel wurden die elektrophoretisch aufgetrennten Proteine auf Nitrocellulosefilter transferiert, fixiert (Towbin et al., Proc. Natl. Acad. Sci. 76 (1979) 4350) und die Immunreaktivität von HIV Fusionsproteinen gegenüber einem Panel von humanen HIV1 bzw. HIV1 und HIV2 Seren ermittelt.

Für das Konstrukt pUC18_HIV2-gp32 wurde in den untersuchten E. coli Wirtsstämmen jeweils eine sehr geringe HIV2-gp32 Expression von 0.1 bis 1% bezogen auf das Gesamtprotein von E. coli gefunden. In Gegenwart des $lacI^q$-Repressorplasmids pFDX500 ergaben sich Expressionswerte von < 0.1%.

Für die Konstruktion pKK233-2/MYYL_HIV2-gp32 wurden die besten Expressionwerte (5 - 10% bezogen auf das E. coli Gesamtprotein) in dem Stamm K165 in Abwesenheit des $lacI^q$-Repressorplasmids gefunden. In Gegenwart des $lacI^q$-Repressorplasmids pFDX500 verringerte sich die Expression auf 2 - 5%.

Die HIV2-gp32 Polypeptide wurden in E. coli ausschließlich als unlösliche Proteinaggregate ("inclusion bodies" "refractile bodies" (RBs); Marston, Biochem. J. 240 (1986) 1-12) synthetisiert, die aus der unlöslichen Zelltrümmerfraktion mit 1xSDS-Probenpuffer bzw. 1xSDS-Probenpuffer mit 6 mol/l Harnstoff gelöst wurden. Die HIV2-gp32 Fusionsproteine reagierten nur mit humanen HIV2 Seren und zeigten keine Kreuzreaktion mit humanen HIV1 Seren in der Westernblot-Analyse.

2.8 Aufreinigung des HIV2-gp32 Polypeptides in größerem Maßstab

Mit dem Plasmid pKK233-2/MYYL_HIV2-gp32 transformierte E. coli K165 Zellen wurden in einem 51-Fermenter bei 30 °C in DYT-Medium mit 50 mg/l Ampicillin und 1% Glucose unter pH-Statisierung (pH 7.5; Inoculum 1% einer stationären Vorkultur) angezogen. Bei Erreichen einer optischen Dichte von 0.8 - 1.0 bei 550 nm wurden die Zellen mit 1 mmol/l IPTG (Endkonzentration) induziert. Nach einer Induktionsphase von 15 Stunden wurden die Zellen (Biomasseausbeute: 20g Naßgewicht/l Fermentationsmedium) durch Zentrifugation geerntet und mit 10 mmol/l Phosphatpuffer, pH 6.8, gewaschen. Die Zellwand wurde mit Lysozym angedaut und anschließend die Zellen mechanisch (Frenchpress) aufgeschlossen. Danach wurden die unlöslichen HIV2-gp32 RBs aus der abzentrifugierten und gewaschenen Zelltrümmerfraktion mit 6 bis 8 mol/l Harnstoff extrahiert (siehe EP-A 0 361 475, Beispiel 1) und anschließend nach üblichen Methoden (Ionenaustauschchromatographie und Gelfiltration in 6 bis 8 mmol/l Harnstoff, Marston et al., Bio/Technology 2 (1984) 800-804; Cabradilla et al., Biotechnology 4 (1986) 128-133) zu > 98% Reinheit aufgereinigt.

**Beispiel 3**

HIV1-gp41 Fusionsproteine

3.1 Plasmid: pUC18_WF113_RsaI/HindIII

In der Konstruktion pUC18_WF113_RsaI/HindIII (siehe Beispiel 1) steht die HIV1-gp41 DNA (RsaI/HindIII-Fragment) unter der Kontrolle des lac Promotors. Die HIV1-gp41 DNA bildet einen offenen Leserahmen mit der lacZ-αPeptid DNA des pUC18 Polylinkers, so daß bei der Expression in E. coli ein lacZ(αPeptid)-HIV1-gp41 Fusionsprotein synthetisiert werden sollte, das N-terminal 17 und C-terminal 83 nicht durch HIV1 DNA kodierte Aminosäuren enthält. Jedoch lag die Expression dieses HIV1-gp41 Polypeptides in den untersuchten E. coli Wirtsstämmen JM109, HB101 und RM82lac$^+$ in Abwesenheit und Anwesenheit von zusätzlichen lacI$^q$-Repressorplasmiden (F'-Episom in JM109 bzw. Cotransformation mit Plasmid pFDX500, siehe Beispiel 2) unter der Nachweisgrenze.

3.2 Konstruktion des Plasmids pKK233-2/MYYL-gp41

In das ca. 4.6 lange pKK233-2/MYYL EcoRI/HindIII-Vektorfragment (Konstruktion pKK233-2/MYYL, siehe Beispiel 2) wurde das ca. 320 Bp lange EcoRI/HindIII-Fragment aus dem Plasmid pUR288__WF113__BamHI/HindIII (siehe Beispiel 1) ligiert. In diesem PLasmid steht die HIV1-gp41 DNA unter der Kontrolle des trc-Promotors. Die HIV1gp41 DNA bildet einen offenen Leserahmen mit der lacY DNA des Polylinkers in dem Plasmid pKK233-2/MYYL, so daß bei der Expression in E. coli ein HIV1-gp41 Fusionsprotein gebildet wird bestehend aus den 4 N-terminalen Aminosäuren MetTyrTyrLeu der Lactosepermease (lacY), 22 konstruktionsbedingten Aminosäuren an der Fusionsstelle, 101 Aminosäuren aus dem envelope gp41 Membranprotein und 12 konstruktionsbedingten Aminosäuren am C-Terminus. Die für dieses Protein kodierende DNA-Sequenz C ist in SEQ ID NO: 11 und die davon abgeleitete Proteinsequenz III in SEQ ID NO: 3 dargestellt.

In E. coli HB101 Wirtszellen, transformiert mit dem Plasmid pKK233-2/MYYL-gp41, wurde nur in Gegenwart des lacI$^q$-Repressorplasmids pFDX500 (Cotransformation) HIV1-gp41 Fusionsprotein synthetisiert (Expressionshöhe: 1 bis 2% bezogen auf das E. coli Gesamtprotein). Die Expressionsanalyse (Zellanzucht, Induktion, SDS-PAGE und Westernblot-Analytik) wurde, wie unter Beispiel 2 beschrieben, durchgeführt.

Überraschenderweise konnte die Expression des HIV1-gp41 Fusionsproteins auf über 20% des E. coli Gesamtproteins gesteigert werden, wenn zusätzlich in die Wirtszelle ein Gen für die in E. coli seltene Arginin-tRNA$^{Arg}$ (Anticodone: AGA, AGG) eingeführt wurde. Dies wurde erreicht durch Cotransformation mit dem dnaY-lacI$^q$-Plasmids pUBS500 ( EP-A 0 368 342) anstelle des Plasmids pFDX500 (supra, siehe Seite 8).

**Beispiel 4**

"Multifunktionelle" HIV Fusionsproteine

Prinzip:

Mittels rekombinanter DNA Technologie wird ein künstliches HIV Fusionsgen hergestellt, das sich aus DNA Teilbereichen mehrerer unterschiedlicher proviraler Genregionen zusammensetzt und einen offenen Leserahmen bildet. Dadurch entsteht ein multifunktionelles HIV Fusionsprotein, das die gewünschten antigenen Bereiche unterschiedlicher retroviraler Proteine eines Virus (z.B aus der gag, pol und env Region von HIV1) und/oder die gewünschten antigenen Bereiche z.B. zweier unterschiedlicher Viren wie HIV1 und HIV2 besitzt. Die Entwicklung multifunktioneller Fusionsantigene erscheint aus Produktionsgründen (Kostenersparnis) sehr attraktiv. Zudem wurde in diesen Fusionsantigenen der Anteil von konstruktionsbedingten Fremdproteinsequenzen weiter reduziert.

4.1 HIV2(envgp32)-HIV1(polp32) Fusionprotein

Konstruktion des Expressionsplasmids pKK233-2/MYYL__gp32__polp32

Das Plasmid pUC18__WF113__INT (siehe Beispiel 1) wurde mit BamHI und BspMI verdaut, die überhängenden 5'-Enden der BamHI- und BspMI-Schnittstelle mit Klenow Polymerase aufgefüllt, das ca. 730 Bp lange BamHI(blunt)/BspMI(blunt)-Fragment isoliert und in die singuläre EcoRV-Schnittstelle des Expressionsvektors pKK233-2/MYYL__HIV2-gp32 (siehe Beispiel 2) ligiert. Die gewünschte Konstruktion, pKK233-2/MYYL__gp32__polp32 (richtige Orientierung des nsertierten BamHI(blunt)/BspMI(blunt)-Fragments), wurde durch Spaltung mit den Restriktionsendonukleasen EcoRI und XbaI ermittelt. Die kodierende DNA Sequenz F ist aus SEQ ID NO: 14 und die Proteinsequenz des HIV2(envgp32)-HIV1(polp32) Fusionsantigens VI aus SEQ ID NO: 6 ersichtlich.

4.2 HIV1(envgp41)-(gagp17-p24-p15) Fusionsprotein

Konstruktion des Expressionsplasmids pKK233-2/MYYL__gp41__p24

Das Plasmid pUC18__WF113__SstI/EcoRI-19 (siehe Beispiel 1) wurde mit BglII verdaut, die überhängenden 5'-Enden der BglII-Schnittstelle mit Klenow Polymerase aufgefüllt, das Plasmid mit PvuII nachgeschnitten, das ca. 960 Bp lange PvuII/BglII(blunt)-Fragment isoliert und in die singuläre HindIII-Schnittstelle des HIV1-gp41 Expressionsvektors pKK233-2/MYYL-gp41 (siehe Beispiel 3) nach Auffüllung der überhängenden 5'-Enden der HindIII-Schnittstelle ligiert. Die gewünschte Konstruktion, pKK233-2/MYYL__gp41__p24 (richtige Orientierung des insertierten PvuII/BglII(blunt)-Fragments) wurde durch Restriktionsanalyse mit den Restriktionsendonukleasen EcoRI und PstI ermittelt. Die kodierende DNA-Sequenz E ist in SEQ ID NO: 13 und die Proteinsequenz V des HIV1(envgp41)-(gagp17-p24-p15) Fusionsproteins in SEQ ID NO: 5 wiedergegeben.

4.3 HIV2(envgp32)-HIV1(polp32-envgp41-gagp17-p24-p15) Fusionsprotein

Konstruktion des Expressionsplasmids pKK233-2/MYYL__gp32__polp32__gp41__p24

Aus dem Plasmid pKK233-2/MYYL__gp32__polp32 (siehe Beispiel 4.1) wurde das ca. 4.7 kBp lange BglII/PvuI-Vektorfragment und aus dem Plasmid pKK233-2/MYYL__gp41__p24 (siehe Beispiel 4.2) wurde das ca. 2.2 kBp lange BamHI/PvuI-Vektorfragment isoliert. Danach wurden die beiden Vektorfragmente ligiert und die gewünschte Plasmidkonstruktion (pKK233-2/MYYL__gp32__polp32__gp41__p24) wurde durch Restriktionsanalyse mit den Restriktionsendonukleasen EcoRI, ApaI und PvuI ermittelt. Die kodierende DNA Sequenz G ist in SEQ ID NO: 15 und die davon abgeleitete Proteinsequenz des HIV2(envgp32)-HIV1-(polp32-envgp41-gagp17-p24-p15) Fusionsproteins VII in SEQ ID NO: 7 wiedergegeben.

Zur Expression der multifunktionellen HIV Fusionsproteine wurden E. coli RM82lac$^+$ Zellen, cotransformiert mit dem dnaY-lacI$^q$ Plasmid pUBS500, benutzt. Die Fusionsproteine wurden bis zu 20% des Gesamtproteins von E. coli synthetisiert, waren unlöslich (RBs) und immunologisch aktiv. Die in den Fusionsproteinen vorhandenen antigenen Determinanten der gag, pol und env Region von HIV1 und der env Region von HIV2 waren alle immunologisch reaktiv. Der Nachweis erfolgte über spezifische monoklonalen Antikörper (gag) und ausgesuchte humane HIV1 (env, pol) und HIV2 (env) Seren. Die Expressionsanalysen (Zellanzucht, Induktion, SDS-PAGE und Westernblot-Analytik) wurden, wie in Beispiel 2 beschrieben, durchgeführt.

**Beispiel 5**

HIV Polypeptid mit Reinigungshilfe

Konstruktion des Plasmids pKK233-2/MYYL gp32__polp32__gp41__p24-polyArgLys

In die siguläre ApaI Schnittstelle des Plasmids pKK233-2/MYYL__gp32__polp32__gp41__p24 wurde ein für 13 basische Aminosäuren (Lys und Arg) codierender Polylinker ligiert.

(ApaI) PvuII (ApaI)

TCTCGTAAAAAGCGTAGACGTAAAAAACGTCGTAAAAAGAAATAGCAGCTGAGGCC

CCGGAGAGCATTTTTCGCATCTGCATTTTTTGCAGCATTTTTCTTTATCGTCGACT

SerArgLysLysArgArgArgLysLysArgArgLysLysLys***

Die Expression des HIV2(envgp32)-HIV1(polp32-envgp41-gagp17-p24-15-poly(Arg-Lys) Fusionsproteins in RM82lac$^+$ Zellen, cotransformiert mit dem dnaY-lacI$^q$ Plasmid pUBS500, und das immunologische Verhalten des Fusionsproteins gegenüber humanen HIV1 und HIV2 Seren entsprach dem HIV2(envgp32)-HIV1(polp32-envgp41-gagp17-p24-p15) Fusionsprotein (vgl. Beispiel 4). Die kodierende DNA-Sequenz H ist aus SEQ ID NO: 16 und die davon abgeleitete Proteinsequenz VIII des HIV2(envgp32)-HIV1(polp32-envgp41-gagp17-p24-15 poly(Arg-Lys) Fusionsproteins aus SEQ ID NO: 8 ersichtlich.

**Beispiel 6**

Immunologische Bestimmung von Anti-HIV-Antikörpern

Zur Beschichtung von Mikrotiterplatten (Dinatech) werden die antigenhaltigen Lösungen in 100 mmol/l Natriumcarbonatpuffer (pH 9,6) auf eine Konzentration von 4 µg/ml verdünnt und 18 Stunden bei Raumtemperatur inkubiert.

Anschließend wird für eine Stunde bei Raumtemperatur mit Rinderserumalbumin (10 mg/ml) in phosphatgepufferter Kochsalzlösung (PBS[2)]) nachinkubiert.

Vor der Inkubation mit Humanserum oder -plasma und zwischen den Testschritten wurde jeweils dreimal mit 0,5 % Tween 20-haltigem demineralisiertem Wasser gewaschen.

Die Probe (Humanserum oder -plasma) wird hundertfach in PBS mit 10 % Kälberserumanteil verdünnt und 2 Stunden bei 37 °C in der beschichteten Mikrotiterplatte inkubiert. Anschließend wird dreimal mit Waschlösung (0,5 % Tween 20 in demineralisiertem Wasser) gewaschen.

Im zweiten Schritt wird mit einem Konjugat aus Peroxidase und polyklonalem Antikörper gegen den Fcγ-Teil von Human IgG (POD-Konjugat) (ca. 30 mU Peroxidase/ml in PBS[2)]) für eine Stunde bei 37 °C inkubiert und dreimal mit Waschlösung gewaschen. Es wird Substratlösung (1,6 mmol/l ABTS® [1)]; 95 mmol/l Phosphat-Citratpuffer, pH 4,4; 3,1 mmol/l Natriumperborat) zugegeben, eine Stunde bei Raumtemperatur inkubiert und die Extinktion bei 492 nm als Maß für die in der Probe vorhandenen spezifischen Antikörper bestimmt. Die Ergebnisse zeigt Tabelle I.

[2)] PBS: 0,15 mol/l Natriumphosphat; 0,9 % NaCl; pH 7,2

[1)] 2,2'Azino-di-[3-ethylbenzthiazolin-sulfonsäure(6)]-diammoniumsalz

T A B E L L E I

Immunologische Reaktivität der unterschiedlichen HIV-Antigene
(ELISA-Ergebnisse [mE(492 nm)]

| Antigen | mit Anti-HIV Poolserum | Normalseren (n = 98)* | rel. Signal |
|---|---|---|---|
| HIV1env-gp41 (Beispiel 3.1) | 1052 | 147±41 | 7,2 |
| HIV1env-gp41 (Beispiel 3.2) | 1308 | 101±16 | 13,0 |
| HIV1gag-p17-p24-p15 | 1758 | 61±20 | 29,0 |
| HIV1pol-p32 | 902 | 98±17 | 9,0 |
| HIV2env-gp32 | 1706 | 121±31 | 14,0 |

* Anzahl der getesteten menschlichen Normalseren

Aus Tabelle I ist ersichtlich, daß die verwendeten HIV1 und HIV2 Fusionsproteine mit den Proteinsequenzen I, II, III und IV (vgl. SEQ ID NO: 1, 2, 3 und 4) keine immunologischen Kreuzreaktionen mit menschlichen Normalseren zeigen.

**Beispiel 7**

Westernblot-Bestimmung (nach Methods in Enzymology 92 (1983) 377 - 391)

Zum Nachweis antigenspezifischer Immunreaktionen werden antikörperhaltige Proben über Nacht bei Raumtemperatur im PBS mit 0,05 % Tween 20 mit den antigentragenden Nitrocellulosestreifen inkubiert.

Nach dreimaligem Waschen wird mit POD-Konjugat (vgl. Beispiel 6) (ca. 50 mU/ml) inkubiert. Nach weiteren drei Waschschritten mit Waschlösung (Beispiel 6) wird 4-Chlor-1-naphthol zugegeben, 15 Minuten bei Raumtemperatur inkubiert und die Farbbildung visuell bestimmt.

Je nach Stärke der Färbung wurde die Immunreaktion mit negativer (-), schwach (+/-), deutlich (+), stark (+ +) und sehr stark (+ + +) eingeteilt. Die Ergebnisse zeigt Tabelle II.

Tabelle II zeigt, daß erfindungsgemäße Fusionsproteine spezifisch mit monoklonalen und polyklonalen Anti-HIV-Antikörpern reagieren.

T A B E L L E II

Bewertung immunologischer Reaktivität im Immunoblot

| Antigen | Monoklonaler Antikörper | | Polyklonaler Antikörper | | | | |
|---|---|---|---|---|---|---|---|
| | Anti gp41 (env) | Anti p24 (gag) | Anti-HIV1 | | | Anti-HIV2 | |
| | | | Probe 1 | Probe 2 | Probe 3 | Probe 1 | Probe 2 |
| HIV1gag-p17 p24-p15 (I) | - | ++ | + | ++ | +/- | + | + |
| HIV1pol-p32 (II) | - | +/- | + | +/- | + | + | +/- |
| HIV1env-gp41 (III) | ++ | - | ++ | + | ++ | - | - |
| HIV2env-gp32 (IV) | - | - | - | - | - | +++ | +++ |
| HIV1(env-gp41)-(gag-p17-p24-p15) (V) | + | ++ | + | ++ | ++ | + | + |
| HIV2(env-gp32)-HIV1(pol-p32) (VI) | - | +/- | + | +/- | + | ++ | ++ |
| HIV2(env-gp32)-HIV1(pol-p32)-(env-gp41)-(gag-p17-p24-p15) (VII) | + | + | +++ | ++ | +++ | ++ | ++ |

- negativ, +/- schwach, + deutlich, ++ stark, +++ sehr stark

**Beispiel 8**

Reagenz zur Bestimmung von Anti-HIV-Antikörpern in Humamserum oder -plasma

Das Reagenz enthält:

Lösung 1:

0,1 bis 1 µg/ml Konjugat aus Antigen und Biotin in Inkubationspuffer (PBS) mit 10 % Kälberserum.

Lösung 2:

POD-Konjugat (Konjugat aus Peroxidase und polyklonalen Antikörpern gegen den Fcγ-Teil von humanem IgG, 30 mU POD/ml) in PBS.

Substratlösung (1,6 mmol/l ABTS®; 95 mmol/l Phosphat-Citratpuffer, pH 4,4; 3,1 mmol/l Natriumperborat)

0,01 ml Probe werden in ein mit Streptavidin beschichtetes Polystyrolröhrchen (Herstellung nach EP-A 0 269 092) gegeben und eine Stunde bei Raumtemperatur mit 1 ml Lösung 1 inkubiert. Nach dreimaligem Waschen mit PBS mit 0,05 % Tween 20 wird eine Stunde bei Raumtemperatur mit 1 ml Lösung 2 inkubiert. Nach dreimaligem Waschen mit PBS mit 0,05 % Tween 20 wird eine Stunde bei Raumtemperatur mit Lösung 2 inkubiert und wiederum dreimal gewaschen. Es wird Substratlösung (1,6 mmol/l ABTS®; 95 mmol/l Phosphat-Citratpuffer, pH 4,4; 3,1 mmol/l Natriumperborat) zugegeben, eine Stunde bei Raumtemperatur inkubiert und die Extinktion bei 492 nm als Maß für die in der Probe vorhandenen spezifischen Antikörper bestimmt.

SEQ ID NO:1 (Aminosäuresequenz I)

ART DER SEQUENZ:Aminosäuresequenz

SEQUENZLÄNGE:337 Aminosäuren

STRANGFORM:Einzelstrang

```
Met Tyr Tyr Leu Glu Phe Pro Asp Lys Gly Asn Ser Ser Gln Val Ser 16
Gln Asn Tyr Pro Ile Val Gln Asn Leu Gln Gly Gln Met Val His Gln 32
Ala Ile Ser Pro Arg Thr Leu Asn Ala Trp Val Lys Val Ile Glu Glu 48
Lys Ala Phe Ser Pro Glu Val Ile Pro Met Phe Ser Ala Leu Ser Glu 64
Gly Ala Thr Pro Gln Asp Leu Asn Thr Met Leu Asn Thr Val Gly Gly 80
His Gln Ala Ala Met Gln Met Leu Lys Glu Thr Ile Asn Glu Glu Ala 96
Ala Glu Trp Asp Arg Val His Pro Val His Ala Gly Pro Ile Ala Pro 112
Gly Gln Met Arg Glu Pro Arg Gly Ser Asp Ile Ala Gly Thr Thr Ser 128
Thr Leu Gln Glu Gln Ile Gly Trp Met Thr Asn Asn Pro Pro Ile Pro 144
Val Gly Glu Ile Tyr Lys Arg Trp Ile Ile Leu Gly Leu Asn Lys Ile 160
Val Arg Met Tyr Ser Pro Val Ser Ile Leu Asp Ile Arg Gln Gly Pro 176
Lys Glu Pro Phe Arg Asp Tyr Val Asp Arg Phe Tyr Lys Thr Leu Arg 192
Ala Glu Gln Ala Ser Gln Glu Val Lys Asn Trp Met Thr Glu Thr Leu 208
Leu Val Gln Asn Ala Asn Pro Asp Cys Lys Thr Ile Leu Lys Ala Leu 224
Gly Pro Ala Ala Thr Leu Glu Glu Met Met Thr Ala Cys Gln Gly Val 240
Gly Gly Pro Gly His Lys Ala Arg Val Leu Ala Glu Ala Met Ser Gln 256
Val Thr Asn Ser Ala Thr Ile Met Met Gln Arg Gly Asn Phe Arg Asn 272
Gln Lys Lys Thr Val Lys Cys Phe Asn Cys Gly Lys Glu Gly His Ile 288
Ala Lys Asn Cys Arg Ala Pro Arg Lys Lys Gly Cys Trp Lys Cys Gly 304
Lys Glu Gly His Gln Met Lys Asp Cys Thr Glu Arg Gln Ala Asn Phe 320
Leu Gly Lys Ile Ser Leu Ala Val Leu Ala Asp Glu Arg Arg Phe Ser 336
Ala
```

SEQ ID NO:2 (Aminosäuresequenz II)
ART DER SEQUENZ:Aminosäuresequenz
SEQUENZLÄNGE:247 Aminosäuren

STRANGFORM:Einzelstrang

```
Met Tyr Tyr Leu Glu Phe Arg Cys Asp Lys Cys Gln Leu Lys Gly Glu 16
Ala Met His Gly Gln Val Asp Cys Ser Pro Gly Ile Trp Gln Leu Asp 32
Cys Thr His Leu Glu Gly Lys Ile Ile Leu Val Ala Val His Val Ala 48
Ser Gly Tyr Ile Glu Ala Glu Val Ile Pro Ala Glu Thr Gly Gln Glu 64
Thr Ala Tyr Phe Ile Leu Lys Leu Ala Gly Arg Trp Pro Val Lys Val 80
Ile His Thr Asp Asn Gly Ser Asn Phe Thr Ser Thr Thr Val Lys Ala 96
Ala Cys Trp Trp Ala Gly Ile Lys Gln Glu Phe Gly Ile Pro Tyr Asn 112
Pro Gln Ser Gln Gly Val Val Glu Ser Met Asn Lys Glu Leu Lys Lys 128
Ile Ile Gly Gln Val Arg Asp Gln Ala Glu His Leu Lys Thr Ala Val 144
Gln Met Ala Val Phe Ile His Asn Phe Lys Arg Lys Gly Gly Ile Gly 160
Gly Tyr Ser Ala Gly Glu Arg Ile Val Asp Ile Ile Ala Thr Asp Ile 176
Gln Thr Lys GluLeu Gln Lys Gln  Ile Ile Lys Ile Gln Asn Phe Arg 192
Val Tyr Tyr Arg Asp Ser Arg Asp Pro Leu Trp Lys Gly Pro Ala Lys 208
Leu Leu Trp Lys Gly Glu Gly Ala Val Val Ile Gln Asp Asn Ser Glu 224
Ile Lys Val Val Pro Arg Arg Lys Ala Lys Ile Ile Arg Asp Tyr Gly 240
Lys His Gly Cys Phe Gly Gly
```

SEQ ID NO:3 (Aminosäuresequenz III)
ART DER SEQUENZ:Aminosäuresequenz
SEQUENZLÄNGE:139 Aminosäuren)

STRANGFORM:Einzelstrang

```
Met Tyr Tyr Leu Glu Phe Gln Leu Ser Ala Gly Arg Tyr His Tyr Gln 16
Leu Val Trp Cys Arg Gly Ser Ser Arg Val Gln Thr Arg Gln Leu Leu 32
Ser Gly Ile Val Gln Gln Gln Asn Asn Leu Leu Arg Ala Ile Glu Thr 48
Gln Gln His Leu Leu Gln Leu Thr Val Trp Gly Ile Lys Gln Leu Gln 64
Ala Arg Val Leu Ala Val Glu Arg Tyr Leu Gln Asp Gln Arg Leu Leu 80
Gly Ile Trp Gly Cys Ser Gly Lys Leu Ile Cys Thr Thr Thr Val Pro 96
Trp Asn Thr Ser Trp Ser Asn Lys Ser Leu Asp Thr Ile Trp His Asn 112
Met Thr Trp Met Glu Trp Glu Arg Glu Ile Asp Asn Tyr Thr Ser Leu 128
Ala Val Leu Ala Asp Glu Arg Arg Phe Ser Ala
```

SEQ ID NO:4 (Aminosäuresequenz IV)

ART DER SEQUENZ:Aminosäuresequenz

SEQUENZLÄNGE:130 Aminosäuren

STRANGFORM:Einzelstrang

Met Tyr Tyr Leu Glu Phe Gln Gln Gln Gln Gln Leu Leu Asp Val Val 16
Lys Arg Gln Gln Glu Leu Leu Arg Leu Thr Val Trp Gly Thr Lys Asn 32
Leu Gln Ala Arg Val Thr Ala Ile Glu Lys Tyr Leu Gln Asp Gln Ala 48
Arg Leu Asn Ser Trp Gly Cys Ala Phe Arg Gln Val Cys His Thr Thr 64
Val Pro Trp Val Asn Asp Ser Leu Ala Pro Asp Trp Asp Asn Met Thr 80
Trp Gln Glu Trp Glu Lys Gln Val Arg Tyr Leu Glu Ala Asn Ile Ser 96
Lys Ser Leu Glu Gln Ala Gln Ile Gln Gln Glu Lys Asn Met Tyr Glu 112
Leu Gln Lys Leu Asn Ser Trp Asp Ile Arg Ser Lys Leu Gly Cys Phe 128
Gly Gly

SEQ ID NO:5 (Aminosäuresequenz V)

ART DER SEQUENZ:Aminosäuresequenz

SEQUENZLÄNGE:458 Aminosäuren

STRANGFORM:Einzelstrang

```
Met Tyr Tyr Leu Glu Phe Gln Leu Ser Ala Gly Arg Tyr His Tyr Gln 16
Leu Val Trp Cys Arg Gly Ser Ser Arg Val Gln Thr Arg Gln Leu Leu 32
Ser Gly Ile Val Gln Gln Gln Asn Asn Leu Leu Arg Ala Ile Glu Thr 48
Gln Gln His Leu Leu Gln Leu Thr Val Trp Gly Ile Lys Gln Leu Gln 64
Ala Arg Val Leu Ala Val Glu Arg Tyr Leu Gln Asp Gln Arg Leu Leu 80
Gly Ile Trp Gly Cys Ser Gly Lys Leu Ile Cys Thr Thr Thr Val Pro 96
Trp Asn Thr Ser Trp Ser Asn Lys Ser Leu Asp Thr Ile Trp His Asn 112
Met Thr Trp Met Glu Trp Glu Arg Glu Ile Asp Asn Tyr Thr Ser Ser 128
Asp Lys Gly Asn Ser Ser Gln Val Ser Gln Asn Tyr Pro Ile Val Gln 144
Asn Leu Gln Gly Gln Met Val His Gln Ala Ile Ser Pro Arg Thr Leu 160
Asn Ala Trp Val Lys Val Ile Glu Glu Lys Ala Phe Ser Pro Glu Val 176
Ile Pro Met Phe Ser Ala Leu Ser Glu Gly Ala Thr Pro Gln Asp Leu 192
Asn Thr Met Leu Asn Thr Val Gly Gly His Gln Ala Ala Met Gln Met 208
Leu Lys Glu Thr Ile Asn Glu Glu Ala Ala Glu Trp Asp Arg Val His 224
Pro Val His Ala Gly Pro Ile Ala Pro Gly Gln Met Arg Glu Pro Arg 240
Gly Ser Asp Ile Ala Gly Thr Thr Ser Thr Leu Gln Glu Gln Ile Gly 256
Trp Met Thr Asn Asn Pro Pro Ile Pro Val Gly Glu Ile Tyr Lys Arg 272
Trp Ile Ile Leu Gly Leu Asn Lys Ile Val Arg Met Tyr Ser Pro Val 288
Ser Ile Leu Asp Ile Arg Gln Gly Pro Lys Glu Pro Phe Arg Asp Tyr 304
Val Asp Arg Phe Tyr Lys Thr Leu Arg Ala Glu Gln Ala Ser Gln Glu 320
Val Lys Asn Trp Met Thr Glu Thr Leu Leu Val Gln Asn Ala Asn Pro 336
Asp Cys Lys Thr Ile Leu Lys Ala Leu Gly Pro Ala Ala Thr Leu Glu 352
Glu Met Met Thr Ala Cys Gln Gly Val Gly Gly Pro Gly His Lys Ala 368
Arg Val Leu Ala Glu Ala Met Ser Gln Val Thr Asn Ser Ala Thr Ile 384
Met Met Gln Arg Gly Asn Phe Arg Asn Gln Lys Lys Thr Val Lys Cys 400
Phe Asn Cys Gly Lys Glu Gly His Ile Ala Lys Asn Cys Arg Ala Pro 416
Arg Lys Lys Gly Cys Trp Lys Cys Gly Lys Glu Gly His Gln Met Lys 432
Asp Cys Thr Glu Arg Gln Ala Asn Phe Leu Gly Lys Ile Ser Leu Ala 448
Val Leu Ala Asp Glu Arg Arg Phe Ser Ala
```

SEQ ID NO:6 (Aminosäuresequenz VI)

ART DER SEQUENZ:Aminosäuresequenz

SEQUENZLÄNGE:376 Aminosäuren

STRANGFORM:Einzelstrang

Met Tyr Tyr Leu Glu Phe Gln Gln Gln Gln Gln Leu Leu Asp Val Val 16
Lys Arg Gln Gln Glu Leu Leu Arg Leu Thr Val Trp Gly Thr Lys Asn 32
Leu Gln Ala Arg Val Thr Ala Ile Glu Lys Tyr Leu Gln Asp Gln Ala 48
Arg Leu Asn Ser Trp Gly Cys Ala Phe Arg Gln Val Cys His Thr Thr 64
Val Pro Trp Val Asn Asp Ser Leu Ala Pro Asp Trp Asp Asn Met Thr 80
Trp Gln Glu Trp Glu Lys Gln Val Arg Tyr Leu Glu Ala Asn Ile Ser 96
Lys Ser Leu Glu Gln Ala Gln Ile Gln Gln Glu Lys Asn Met Tyr Glu 112
Leu Gln Lys Leu Asn Ser Trp Asp Asp Pro Leu Glu Ser Cys Asp Lys 128
Cys Gln Leu Lys Gly Glu Ala Met His Gly Gln Val Asp Cys Ser Pro 144
Gly Ile Trp Gln Leu Asp Cys Thr His Leu Glu Gly Lys Ile Ile Leu 160
Val Ala Val His Val Ala Ser Gly Tyr Ile Glu Ala Glu Val Ile Pro 176
Ala Glu Thr Gly Gln Glu Thr Ala Tyr Phe Ile Leu Lys Leu Ala Gly 192
Arg Trp Pro Val Lys Val Ile His Thr Asp Asn Gly Ser Asn Phe Thr 208
Ser Thr Thr Val Lys Ala Ala Cys Trp Trp Ala Gly Ile Lys Gln Glu 224
Phe Gly Ile Pro Tyr Asn Pro Gln Ser Gln Gly Val Val Glu Ser Met 240
Asn Lys Glu Leu Lys Lys Ile Ile Gly Gln Val Arg Asp Gln Ala Glu 256
His Leu Lys Thr Ala Val Gln Met Ala Val Phe Ile His Asn Phe Lys 272
Arg Lys Gly Gly Ile Gly Gly Tyr Ser Ala Gly Glu Arg Ile Val Asp 288
Ile Ile Ala Thr Asp Ile Gln Thr Lys Glu Leu Gln Lys Gln Ile Ile 304
Lys Ile Gln Asn Phe Arg Val Tyr Tyr Arg Asp Ser Arg Asp Pro Leu 320
Trp Lys Gly Pro Ala Lys Leu Leu Trp Lys Gly Glu Gly Ala Val Val 336
Ile Gln Asp Asn Ser Glu Ile Lys Val Val Pro Arg Arg Lys Ala Lys 352
Ile Ile Arg Asp Tyr Gly Lys Gln Ser Asp Leu Ser Phe Pro Val Leu 368
Ala Asp Glu Arg Gly Phe Ser Ala

SEQ ID NO:7 (Aminosäuresequenz VII)

ART DER SEQUENZ:Aminosäuresequenz

SEQUENZLÄNGE:799 Aminosäuren

STRANGFORM:Einzelstrang

Met Tyr Tyr Leu Glu Phe Gln Gln Gln Gln Gln Leu Leu Asp Val Val 16
Lys Arg Gln Gln Glu Leu Leu Arg Leu Thr Val Trp Gly Thr Lys Asn 32
Leu Gln Ala Arg Val Thr Ala Ile Glu Lys Tyr Leu Gln Asp Gln Ala 48
Arg Leu Asn Ser Trp Gly Cys Ala Phe Arg Gln Val Cys His Thr Thr 64
Val Pro Trp Val Asn Asp Ser Leu Ala Pro Asp Trp Asp Asn Met Thr 80
Trp Gln Glu Trp Glu Lys Gln Val Arg Tyr Leu Glu Ala Asn Ile Ser 96
Lys Ser Leu Glu Gln Ala Gln Ile Gln Gln Glu Lys Asn Met Tyr Glu 112
Leu Gln Lys Leu Asn Ser Trp Asp Asp Pro Leu Glu Ser Cys Asp Lys 128
Cys Gln Leu Lys Gly Glu Ala Met His Gly Gln Val Asp Cys Ser Pro 144
Gly Ile Trp Gln Leu Asp Cys Thr His Leu Glu Gly Lys Ile Ile Leu 160
Val Ala Val His Val Ala Ser Gly Tyr Ile Glu Ala Glu Val Ile Pro 176
Ala Glu Thr Gly Gln Glu Thr Ala Tyr Phe Ile Leu Lys Leu Ala Gly 192
Arg Trp Pro Val Lys Val Ile His Thr Asp Asn Gly Ser Asn Phe Thr 208
Ser Thr Thr Val Lys Ala Ala Cys Trp Trp Ala Gly Ile Lys Gln Glu 224
Phe Gly Ile Pro Tyr Asn Pro Gln Ser Gln Gly Val Val Glu Ser Met 240
Asn Lys Glu Leu Lys Lys Ile Ile Gly Gln Val Arg Asp Gln Ala Glu 256
His Leu Lys Thr Ala Val Gln Met Ala Val Phe Ile His Asn Phe Lys 272
Arg Lys Gly Gly Ile Gly Gly Tyr Ser Ala Gly Glu Arg Ile Val Asp 288
Ile Ile Ala Thr Asp Ile Gln Thr Lys Glu Leu Gln Lys Gln Ile Ile 304
Lys Ile Gln Asn Phe Arg Val Tyr Tyr Arg Asp Ser Arg Asp Pro Leu 320
Trp Lys Gly Pro Ala Lys Leu Leu Trp Lys Gly Glu Gly Ala Val Val 336
Ile Gln Asp Asn Ser Glu Ile Lys Val Val Pro Arg Arg Lys Ala Lys 352
Ile Ile Arg Asp Tyr Gly Lys Gln Ser Asp Arg Ser Ser Arg Val Gln 368
Thr Arg Gln Leu Leu Ser Gly Ile Val Gln Gln Gln Asn Asn Leu Leu 384
Arg Ala Ile Glu Thr Gln Gln His Leu Leu Gln Leu Thr Val Trp Gly 400
Ile Lys Gln Leu Gln Ala Arg Val Leu Ala Val Glu Arg Tyr Leu Gln 416
Asp Gln Arg Leu Leu Gly Ile Trp Gly Cys Ser Gly Lys Leu Ile Cys 432

Thr Thr Thr Val Pro Trp Asn Thr Ser Trp Ser Asn Lys Ser Leu Asp 448
Thr Ile Trp His Asn Met Thr Trp Met Glu Trp Glu Arg Glu Ile Asp 464
Asn Tyr Thr Ser Ser Asp Lys Gly Asn Ser Ser Gln Val Ser Gln Asn 480
Tyr Pro Ile Val Gln Asn Leu Gln Gly Gln Met Val His Gln Ala Ile 496
Ser Pro Arg Thr Leu Asn Ala Trp Val Lys Val Ile Glu Glu Lys Ala 512
Phe Ser Pro Glu Val Ile Pro Met Phe Ser Ala Leu Ser Glu Gly Ala 528
Thr Pro Gln Asp Leu Asn Thr Met Leu Asn Thr Val Gly Gly His Gln 544
Ala Ala Met Gln Met Leu Lys Glu Thr Ile Asn Glu Glu Ala Ala Glu 560
Trp Asp Arg Val His Pro Val His Ala Gly Pro Ile Ala Pro Gly Gln 576
Met Arg Glu Pro Arg Gly Ser Asp Ile Ala Gly Thr Thr Ser Thr Leu 592
Gln Glu Gln Ile Gly Trp Met Thr Asn Asn Pro Pro Ile Pro Val Gly 608
Glu Ile Tyr Lys Arg Trp Ile Ile Leu Gly Leu Asn Lys Ile Val Arg 624
Met Tyr Ser Pro Val Ser Ile Leu Asp Ile Arg Gln Gly Pro Lys Glu 640
Pro Phe Arg Asp Tyr Val Asp Arg Phe Tyr Lys Thr Leu Arg Ala Glu 656
Gln Ala Ser Gln Glu Val Lys Asn Trp Met Thr Glu Thr Leu Leu Val 672
Gln Asn Ala Asn Pro Asp Cys Lys Thr Ile Leu Lys Ala Leu Gly Pro 688
Ala Ala Thr Leu Glu Glu Met Met Thr Ala Cys Gln Gly Val Gly Gly 704
Pro Gly His Lys Ala Arg Val Leu Ala Glu Ala Met Ser Gln Val Thr 720
Asn Ser Ala Thr Ile Met Met Gln Arg Gly Asn Phe Arg Asn Gln Lys 736
Lys Thr Val Lys Cys Phe Asn Cys Gly Lys Glu Gly His Ile Ala Lys 752
Asn Cys Arg Ala Pro Arg Lys Lys Gly Cys Trp Lys Cys Gly Lys Glu 768
Gly His Gln Met Lys Asp Cys Thr Glu Arg Gln Ala Asn Phe Leu Gly 784
Lys Ile Ser Leu Ala Val Leu Ala Asp Glu Arg Arg Phe Ser Ala

SEQ ID NO:8 (Aminosäuresequenz VIII)

ART DER SEQUENZ:Aminosäuresequenz

SEQUENZLÄNGE:770 Aminosäuren

STRANGFORM:Einzelstrang

Met Tyr Tyr Leu Glu Phe Gln Gln Gln Gln Gln Leu Leu Asp Val Val 16
Lys Arg Gln Gln Glu Leu Leu Arg Leu Thr Val Trp Gly Thr Lys Asn 32
Leu Gln Ala Arg Val Thr Ala Ile Glu Lys Tyr Leu Gln Asp Gln Ala 48
Arg Leu Asn Ser Trp Gly Cys Ala Phe Arg Gln Val Cys His Thr Thr 64
Val Pro Trp Val Asn Asp Ser Leu Ala Pro Asp Trp Asp Asn Met Thr 80
Trp Gln Glu Trp Glu Lys Gln Val Arg Tyr Leu Glu Ala Asn Ile Ser 96
Lys Ser Leu Glu Gln Ala Gln Ile Gln Gln Glu Lys Asn Met Tyr Glu 112
Leu Gln Lys Leu Asn Ser Trp Asp Asp Pro Leu Glu Ser Cys Asp Lys 128
Cys Gln Leu Lys Gly Glu Ala Met His Gly Gln Val Asp Cys Ser Pro 144
Gly Ile Trp Gln Leu Asp Cys Thr His Leu Glu Gly Lys Ile Ile Leu 160
Val Ala Val His Val Ala Ser Gly Tyr Ile Glu Ala Glu Val Ile Pro 176
Ala Glu Thr Gly Gln Glu Thr Ala Tyr Phe Ile Leu Lys Leu Ala Gly 192
Arg Trp Pro Val Lys Val Ile His Thr Asp Asn Gly Ser Asn Phe Thr 208
Ser Thr Thr Val Lys Ala Ala Cys Trp Trp Ala Gly Ile Lys Gln Glu 224
Phe Gly Ile Pro Tyr Asn Pro Gln Ser Gln Gly Val Val Glu Ser Met 240
Asn Lys Glu Leu Lys Lys Ile Ile Gly Gln Val Arg Asp Gln Ala Glu 256
His Leu Lys Thr Ala Val Gln Met Ala Val Phe Ile His Asn Phe Lys 272
Arg Lys Gly Gly Ile Gly Gly Tyr Ser Ala Gly Glu Arg Ile Val Asp 288
Ile Ile Ala Thr Asp Ile Gln Thr Lys Glu Leu Gln Lys Gln Ile Ile 304
Lys Ile Gln Asn Phe Arg Val Tyr Tyr Arg Asp Ser Arg Asp Pro Leu 320
Trp Lys Gly Pro Ala Lys Leu Leu Trp Lys Gly Glu Gly Ala Val Val 336
Ile Gln Asp Asn Ser Glu Ile Lys Val Val Pro Arg Arg Lys Ala Lys 352
Ile Ile Arg Asp Tyr Gly Lys Gln Ser Asp Arg Ser Ser Arg Val Gln 368
Thr Arg Gln Leu Leu Ser Gly Ile Val Gln Gln Gln Asn Asn Leu Leu 384
Arg Ala Ile Glu Thr Gln Gln His Leu Leu Gln Leu Thr Val Trp Gly 400
Ile Lys Gln Leu Gln Ala Arg Val Leu Ala Val Glu Arg Tyr Leu Gln 416

Asp Gln Arg Leu Leu Gly Ile Trp Gly Cys Ser Gly Lys Leu Ile Cys 432
Thr Thr Thr Val Pro Trp Asn Thr Ser Trp Ser Asn Lys Ser Leu Asp 448
Thr Ile Trp His Asn Met Thr Trp Met Glu Trp Glu Arg Glu Ile Asp 464
Asn Tyr Thr Ser Ser Asp Lys Gly Asn Ser Ser Gln Val Ser Gln Asn 480
Tyr Pro Ile Val Gln Asn Leu Gln Gly Gln Met Val His Gln Ala Ile 496
Ser Pro Arg Thr Leu Asn Ala Trp Val Lys Val Ile Glu Glu Lys Ala 512
Phe Ser Pro Glu Val Ile Pro Met Phe Ser Ala Leu Ser Glu Gly Ala 528
Thr Pro Gln Asp Leu Asn Thr Met Leu Asn Thr Val Gly Gly His Gln 544
Ala Ala Met Gln Met Leu Lys Glu Thr Ile Asn Glu Glu Ala Ala Glu 560
Trp Asp Arg Val His Pro Val His Ala Gly Pro Ile Ala Pro Gly Gln 576
Met Arg Glu Pro Arg Gly Ser Asp Ile Ala Gly Thr Thr Ser Thr Leu 592
Gln Glu Gln Ile Gly Trp Met Thr Asn Asn Pro Pro Ile Pro Val Gly 608
Glu Ile Tyr Lys Arg Trp Ile Ile Leu Gly Leu Asn Lys Ile Val Arg 624
Met Tyr Ser Pro Val Ser Ile Leu Asp Ile Arg Gln Gly Pro Lys Glu 640
Pro Phe Arg Asp Tyr Val Asp Arg Phe Tyr Lys Thr Leu Arg Ala Glu 656
Gln Ala Ser Gln Glu Val Lys Asn Trp Met Thr Glu Thr Leu Leu Val 672
Gln Asn Ala Asn Pro Asp Cys Lys Thr Ile Leu Lys Ala Leu Gly Pro 688
Ala Ala Thr Leu Glu Glu Met Met Thr Ala Cys Gln Gly Val Gly Gly 704
Pro Gly His Lys Ala Arg Val Leu Ala Glu Ala Met Ser Gln Val Thr 720
Asn Ser Ala Thr Ile Met Met Gln Arg Gly Asn Phe Arg Asn Gln Lys 736
Lys Thr Val Lys Cys Phe Asn Cys Gly Lys Glu Gly His Ile Ala Lys 752
Asn Cys Arg Ala Ser Arg Lys Lys Arg Arg Arg Lys Lys Arg Arg Lys 768
Lys Lys

SEQ ID NO:9 (Nukleinsäuresequenz A)
ART DER SEQUENZ:Nukleotidsequenz
SEQUENZLÄNGE:1014 Basenpaare

STRANGFORM:Einzelstrang

```
ATGTACTATT TAGAATTCCC TGACAAAGGA AACAGCAGCC AGGTCAGTCA AAATTACCCT     60
ATAGTGCAGA ACCTACAGGG GCAAATGGTA CATCAGGCCA TATCACCTAG AACTTTAAAT    120
GCATGGGTAA AAGTAATAGA AGAAAAGGCT TTCAGCCCAG AAGTGATACC CATGTTTTCA    180
GCATTATCAG AAGGAGCCAC CCCACAAGAT TTAAATACCA TGCTAAACAC AGTGGGGGGA    240
CATCAAGCAG CCATGCAAAT GTTAAAAGAG ACCATCAATG AGGAAGCTGC AGAATGGGAT    300
AGAGTGCATC CAGTGCATGC AGGGCCTATT GCACCAGGCC AGATGAGAGA ACCAAGGGGA    360
AGTGATATAG CAGGAACTAC TAGTACCCTT CAGGAACAAA TAGGATGGAT GACAAACAAT    420
CCACCTATCC CAGTAGGAGA AATCTATAAA AGATGGATAA TCCTGGGATT AAATAAAATA    480
GTAAGAATGT ATAGTCCTGT TAGTATTCTG GACATAAGAC AAGGACCAAA GGAACCCTTT    540
AGAGACTATG TAGATCGGTT CTATAAAACT TTAAGAGCCG AGCAAGCTTC ACAGGAGGTA    600
AAAAATTGGA TGACAGAAAC CTTGTTGGTC CAAAATGCGA ACCCAGACTG TAAGACTATT    660
CTAAAAGCAT TAGGACCAGC AGCTACACTA GAAGAAATGA TGACAGCATG TCAGGGAGTG    720
GGGGGACCCG GCCATAAGGC AAGAGTGTTG GCTGAAGCAA TGAGCCAAGT AACAAATTCA    780
GCTACCATAA TGATGCAGAG AGGTAATTTT AGGAACCAAA AAAAAACTGT TAAGTGTTTC    840
AATTGTGGCA AAGAAGGGCA CATAGCCAAA AATTGCAGGG CCCCTAGGAA AAAGGGCTGT    900
TGGAAATGTG GAAAGGAAGG ACATCAAATG AAAGATTGTA CTGAGAGACA GGCTAATTTT    960
TTAGGGAAGA TCAGCTTGGC TGTTTTGGCG GATGAGAGAA GATTTTCAGC CTGA         1014
```

SEQ ID NO:10 (Nukleinsäuresequenz B)
ART DER SEQUENZ:Nukleotidsequenz
SEQUENZLÄNGE:744 Basenpaare

STRANGFORM:Einzelstrang

```
ATGTACTATT TAGAATTCCG CTGTGATAAA TGTCAGCTAA AAGGAGAAGC CATGCATGGA    60
CAAGTAGATT GTAGTCCAGG AATATGGCAA CTAGATTGCA CACATTTAGA AGGAAAAATT   120
ATCCTGGTAG CAGTTCATGT AGCCAGTGGC TATATAGAAG CAGAAGTTAT TCCAGCAGAG   180
ACAGGGCAGG AAACAGCATA CTTTATCTTA AAATTAGCAG GAAGATGGCC AGTAAAAGTA   240
ATACATACAG ACAATGGCAG TAATTTCACC AGTACTACGG TTAAGGCCGC CTGTTGGTGG   300
GCGGGGATCA AGCAGGAATT TGGCATTCCC TACAATCCCC AAAGTCAAGG AGTAGTAGAA   360
TCTATGAATA AAGAATTAAA GAAAATTATA GGACAGGTAA GAGATCAGGC TGAACATCTT   420
AAGACAGCAG TACAAATGGC AGTATTTATC CACAATTTTA AAAGAAAAGG GGGGATTGGG   480
GGGTACAGTG CAGGGGAAAG AATAGTAGAC ATAATAGCAA CAGACATACA AACTAAAGAA   540
TTACAAAAAC AAATTATAAA AATTCAAAAT TTTCGGGTTT ATTACAGGGA CAGCAGAGAT   600
CCACTTTGGA AAGGACCAGC AAAGCTCCTC TGGAAAGGTG AAGGGGCAGT AGTAATACAA   660
GACAATAGTG AAATAAAAGT AGTGCCAAGG AGAAAAGCAA AGATCATTAG GGATTATGGA   720
AAACATGGCT GTTTTGGCGG ATGA                                          744
```

SEQ ID NO:11 (Nukleinsäuresequenz C)
ART DER SEQUENZ:Nukleotidsequenz
SEQUENZLÄNGE:420 Basenpaare

STRANGFORM:Einzelstrang

```
ATGTACTATT TAGAATTCCA GCTGAGCGCC GGTCGCTACC ATTACCAGTT GGTCTGGTGT    60
CGGGGATCCT CTAGAGTCCA GACCAGACAA TTATTGTCTG GAATAGTGCA ACAGCAGAAC   120
AATTTGCTGA GGGCTATTGA GACGCAACAA CATCTGTTGC AACTCACGGT CTGGGGCATC   180
AAACAGCTCC AGGCAAGAGT CCTGGCTGTG GAAAGATACC TACAGGATCA ACGGCTCCTA   240
GGGATTTGGG GTTGCTCTGG AAAACTCATC TGCACCACTA CTGTGCCTTG GAACACTAGT   300
TGGAGTAATA AATCTCTAGA TACAATTTGG CATAACATGA CCTGGATGGA GTGGGAAAGA   360
GAAATTGACA ATTACACAAG CTTGGCTGTT TTGGCGGATG AGAGAAGATT TTCAGCCTGA   420
```

SEQ ID NO:12 (Nukleinsäuresequenz D)

ART DER SEQUENZ:Nukleotidsequenz

SEQUENZLÄNGE:393 Basenpaare

STRANGFORM:Einzelstrang

```
ATGTACTATT TAGAATTCCA ACAGCAACAG CAGTTGTTGG ACGTTGTTAA ACGTCAACAG    60
GAACTGTTGC GTCTGACCGT TTGGGGAACC AAGAACCTTC AGGCTAGAGT TACCGCTATC   120
GAAAAATACC TTCAAGACCA GGCTCGTTTG AACTCCTGGG GTTGCGCTTT TAGACAGGTT   180
TGTCATACCA CGGTACCGTG GGTTAACGAC TCTCTGGCTC CAGACTGGGA CAACATGACC   240
TGGCAGGAAT GGGAAAAGCA AGTTCGTTAC TTGGAAGCTA ACATCTCCAA ATCTCTGGAA   300
CAGGCTCAAA TCCAGCAAGA AAAAAACATG TACGAACTGC AGAAGTTGAA CTCTTGGGAT   360
ATCAGATCTA AGCTTGGCTG TTTTGGCGGA TGA                                393
```

SEQ ID NO:13 (Nukleinsäuresequenz E)

ART DER SEQUENZ:Nukleotidsequenz

SEQUENZLÄNGE:1377 Basenpaare

STRANGFORM:Einzelstrang

```
ATGTACTATT TAGAATTCCA GCTGAGCGCC GGTCGCTACC ATTACCAGTT GGTCTGGTGT   60
CGGGGATCCT CTAGAGTCCA GACCAGACAA TTATTGTCTG GAATAGTGCA ACAGCAGAAC  120
AATTTGCTGA GGGCTATTGA GACGCAACAA CATCTGTTGC AACTCACGGT CTGGGGCATC  180
AAACAGCTCC AGGCAAGAGT CCTGGCTGTG GAAAGATACC TACAGGATCA ACGGCTCCTA  240
GGGATTTGGG GTTGCTCTGG AAAACTCATC TGCACCACTA CTGTGCCTTG GAACACTAGT  300
TGGAGTAATA AATCTCTAGA TACAATTTGG CATAACATGA CCTGGATGGA GTGGGAAAGA  360
GAAATTGACA ATTACACAAG CTCTGACAAA GGAAACAGCA GCCAGGTCAG TCAAAATTAC  420
CCTATAGTGC AGAACCTACA GGGGCAAATG GTACATCAGG CCATATCACC TAGAACTTTA  480
AATGCATGGG TAAAAGTAAT AGAAGAAAAG GCTTTCAGCC CAGAAGTGAT ACCCATGTTT  540
TCAGCATTAT CAGAAGGAGC CACCCCACAA GATTTAAATA CCATGCTAAA CACAGTGGGG  600
GGACATCAAG CAGCCATGCA AATGTTAAAA GAGACCATCA ATGAGGAAGC TGCAGAATGG  660
GATAGAGTGC ATCCAGTGCA TGCAGGGCCT ATTGCACCAG GCCAGATGAG AGAACCAAGG  720
GGAAGTGATA TAGCAGGAAC TACTAGTACC CTTCAGGAAC AAATAGGATG GATGACAAAC  780
AATCCACCTA TCCCAGTAGG AGAAATCTAT AAAAGATGGA TAATCCTGGG ATTAAATAAA  840
ATAGTAAGAA TGTATAGTCC TGTTAGTATT CTGGACATAA GACAAGGACC AAAGGAACCC  900
TTTAGAGACT ATGTAGATCG GTTCTATAAA ACTTTAAGAG CCGAGCAAGC TTCACAGGAG  960
GTAAAAAATT GGATGACAGA AACCTTGTTG GTCCAAAATG CGAACCCAGA CTGTAAGACT 1020
ATTCTAAAAG CATTAGGACC AGCAGCTACA CTAGAAGAAA TGATGACAGC ATGTCAGGGA 1080
GTGGGGGGAC CCGGCCATAA GGCAAGAGTG TTGGCTGAAG CAATGAGCCA AGTAACAAAT 1140
TCAGCTACCA TAATGATGCA GAGAGGTAAT TTTAGGAACC AAAAAAAAAC TGTTAAGTGT 1200
TTCAATTGTG GCAAAGAAGG GCACATAGCC AAAAATTGCA GGGCCCCTAG GAAAAAGGGC 1260
TGTTGGAAAT GTGGAAAGGA AGGACATCAA ATGAAAGATT GTACTGAGAG ACAGGCTAAT 1320
TTTTTAGGGA AGATCAGCTT GGCTGTTTTG GCGGATGAGA GAAGATTTTC AGCCTGA    1377
```

SEQ ID NO:14 (Nukleinsäuresequenz F)

ART DER SEQUENZ:Nukleotidsequenz

SEQUENZLÄNGE:1131 Basenpaare

STRANGFORM:Einzelstrang

ATGTACTATT TAGAATTCCA ACAGCAACAG CAGTTGTTGG ACGTTGTTAA ACGTCAACAG 60

GAACTGTTGC GTCTGACCGT TTGGGGAACC AAGAACCTTC AGGCTAGAGT TACCGCTATC 120

GAAAAATACC TTCAAGACCA GGCTCGTTTG AACTCCTGGG GTTGCGCTTT TAGACAGGTT 180

TGTCATACCA CGGTACCGTG GGTTAACGAC TCTCTGGCTC CAGACTGGGA CAACATGACC 240

TGGCAGGAAT GGGAAAAGCA AGTTCGTTAC TTGGAAGCTA ACATCTCCAA ATCTCTGGAA 300

CAGGCTCAAA TCCAGCAAGA AAAAAACATG TACGAACTGC AGAAGTTGAA CTCTTGGGAT 360

GATCCTCTAG AGTCCTGTGA TAAATGTCAG CTAAAAGGAG AAGCCATGCA TGGACAAGTA 420

GATTGTAGTC CAGGAATATG GCAACTAGAT TGCACACATT TAGAAGGAAA AATTATCCTG 480

GTAGCAGTTC ATGTAGCCAG TGGCTATATA GAAGCAGAAG TTATTCCAGC AGAGACAGGG 540

CAGGAAACAG CATACTTTAT CTTAAAATTA GCAGGAAGAT GGCCAGTAAA AGTAATACAT 600

ACAGACAATG GCAGTAATTT CACCAGTACT ACGGTTAAGG CCGCCTGTTG GTGGGCGGGG 660

ATCAAGCAGG AATTTGGCAT TCCCTACAAT CCCCAAAGTC AAGGAGTAGT AGAATCTATG 720

AATAAAGAAT TAAAGAAAAT TATAGGACAG GTAAGAGATC AGGCTGAACA TCTTAAGACA 780

GCAGTACAAA TGGCAGTATT TATCCACAAT TTTAAAAGAA AAGGGGGGAT TGGGGGGTAC 840

AGTGCAGGGG AAAGAATAGT AGACATAATA GCAACAGACA TACAAACTAA AGAATTACAA 900

AAACAAATTA TAAAAATTCA AAATTTTCGG GTTTATTACA GGGACAGCAG AGATCCACTT 960

TGGAAAGGAC CAGCAAAGCT CCTCTGGAAA GGTGAAGGGG CAGTAGTAAT ACAAGACAAT 1020

AGTGAAATAA AAGTAGTGCC AAGGAGAAAA GCAAAGATCA TTAGGGATTA TGGAAAACAA 1080

TCAGATCTAA GCTTCCCTGT TTTGGCGGAT GAGAGAGGAT TTTCAGCCTG A 1131

SEQ ID NO:15 (Nukleinsäuresequenz G)

ART DER SEQUENZ:Nukleotidsequenz

SEQUENZLÄNGE:2400 Basenpaare

STRANGFORM:Einzelstrang

```
ATGTACTATT TAGAATTCCA ACAGCAACAG CAGTTGTTGG ACGTTGTTAA ACGTCAACAG    60
GAACTGTTGC GTCTGACCGT TTGGGGAACC AAGAACCTTC AGGCTAGAGT TACCGCTATC   120
GAAAAATACC TTCAAGACCA GGCTCGTTTG AACTCCTGGG GTTGCGCTTT TAGACAGGTT   180
TGTCATACCA CGGTACCGTG GGTTAACGAC TCTCTGGCTC CAGACTGGGA CAACATGACC   240
TGGCAGGAAT GGGAAAAGCA AGTTCGTTAC TTGGAAGCTA ACATCTCCAA ATCTCTGGAA   300
CAGGCTCAAA TCCAGCAAGA AAAAAACATG TACGAACTGC AGAAGTTGAA CTCTTGGGAT   360
GATCCTCTAG AGTCCTGTGA TAAATGTCAG CTAAAAGGAG AAGCCATGCA TGGACAAGTA   420
GATTGTAGTC CAGGAATATG GCAACTAGAT TGCACACATT TAGAAGGAAA AATTATCCTG   480
GTAGCAGTTC ATGTAGCCAG TGGCTATATA GAAGCAGAAG TTATTCCAGC AGAGACAGGG   540
CAGGAAACAG CATACTTTAT CTTAAAATTA GCAGGAAGAT GGCCAGTAAA AGTAATACAT   600
ACAGACAATG GCAGTAATTT CACCAGTACT ACGGTTAAGG CCGCCTGTTG GTGGGCGGGG   660
ATCAAGCAGG AATTTGGCAT TCCCTACAAT CCCCAAAGTC AAGGAGTAGT AGAATCTATG   720
AATAAAGAAT TAAAGAAAAT TATAGGACAG GTAAGAGATC AGGCTGAACA TCTTAAGACA   780
GCAGTACAAA TGGCAGTATT TATCCACAAT TTTAAAAGAA AAGGGGGGAT TGGGGGGTAC   840
AGTGCAGGGG AAAGAATAGT AGACATAATA GCAACAGACA TACAAACTAA AGAATTACAA   900
AAACAAATTA TAAAAATTCA AAATTTTCGG GTTTATTACA GGGACAGCAG AGATCCACTT   960
TGGAAAGGAC CAGCAAAGCT CCTCTGGAAA GGTGAAGGGG CAGTAGTAAT ACAAGACAAT  1020
AGTGAAATAA AAGTAGTGCC AAGGAGAAAA GCAAAGATCA TTAGGGATTA TGGAAAACAA  1080
TCAGATCGAT CCTCTAGAGT CCAGACCAGA CAATTATTGT CTGGAATAGT GCAACAGCAG  1140
AACAATTTGC TGAGGGCTAT TGAGACGCAA CAACATCTGT TGCAACTCAC GGTCTGGGGC  1200
ATCAAACAGC TCCAGGCAAG AGTCCTGGCT GTGGAAAGAT ACCTACAGGA TCAACGGCTC  1260
CTAGGGATTT GGGGTTGCTC TGGAAAACTC ATCTGCACCA CTACTGTGCC TTGGAACACT  1320
AGTTGGAGTA ATAAATCTCT AGATACAATT TGGCATAACA TGACCTGGAT GGAGTGGGAA  1380
AGAGAAATTG ACAATTACAC AAGCTCTGAC AAAGGAAACA GCAGCCAGGT CAGTCAAAAT  1440
TACCCTATAG TGCAGAACCT ACAGGGGCAA ATGGTACATC AGGCCATATC ACCTAGAACT  1500
```

```
TTAAATGCAT GGGTAAAAGT AATAGAAGAA AAGGCTTTCA GCCCAGAAGT GATACCCATG  1560
TTTTCAGCAT TATCAGAAGG AGCCACCCCA CAAGATTTAA ATACCATGCT AAACACAGTG  1620
GGGGGACATC AAGCAGCCAT GCAAATGTTA AAAGAGACCA TCAATGAGGA AGCTGCAGAA  1680
TGGGATAGAG TGCATCCAGT GCATGCAGGG CCTATTGCAC CAGGCCAGAT GAGAGAACCA  1740
AGGGGAAGTG ATATAGCAGG AACTACTAGT ACCCTTCAGG AACAAATAGG ATGGATGACA  1800
AACAATCCAC CTATCCCAGT AGGAGAAATC TATAAAAGAT GGATAATCCT GGGATTAAAT  1860
AAAATAGTAA GAATGTATAG TCCTGTTAGT ATTCTGGACA TAAGACAAGG ACCAAAGGAA  1920
CCCTTTAGAG ACTATGTAGA TCGGTTCTAT AAAACTTTAA GAGCCGAGCA AGCTTCACAG  1980
GAGGTAAAAA ATTGGATGAC AGAAACCTTG TTGGTCCAAA ATGCGAACCC AGACTGTAAG  2040
ACTATTCTAA AAGCATTAGG ACCAGCAGCT ACACTAGAAG AAATGATGAC AGCATGTCAG  2100
GGAGTGGGGG GACCCGGCCA TAAGGCAAGA GTGTTGGCTG AAGCAATGAG CCAAGTAACA  2160
AATTCAGCTA CCATAATGAT GCAGAGAGGT AATTTTAGGA ACCAAAAAAA AACTGTTAAG  2220
TGTTTCAATT GTGGCAAAGA AGGGCACATA GCCAAAAATT GCAGGGCCCC TAGGAAAAAG  2280
GGCTGTTGGA AATGTGGAAA GGAAGGACAT CAAATGAAAG ATTGTACTGA GAGACAGGCT  2340
AATTTTTTAG GGAAGATCAG CTTGGCTGTT TTGGCGGATG AGAGAAGATT TTCAGCCTGA  2400
```

SEQ ID NO:16 (Nukleinsäuresequenz H)

ART DER SEQUENZ:Nukleotidsequenz

SEQUENZLÄNGE:2313 Basenpaare

STRANGFORM:Einzelstrang

```
ATGTACTATT TAGAATTCCA ACAGCAACAG CAGTTGTTGG ACGTTGTTAA ACGTCAACAG   60
GAACTGTTGC GTCTGACCGT TTGGGGAACC AAGAACCTTC AGGCTAGAGT TACCGCTATC  120
GAAAAATACC TTCAAGACCA GGCTCGTTTG AACTCCTGGG GTTGCGCTTT TAGACAGGTT  180
TGTCATACCA CGGTACCGTG GGTTAACGAC TCTCTGGCTC CAGACTGGGA CAACATGACC  240
TGGCAGGAAT GGGAAAAGCA AGTTCGTTAC TTGGAAGCTA ACATCTCCAA ATCTCTGGAA  300
CAGGCTCAAA TCCAGCAAGA AAAAAACATG TACGAACTGC AGAAGTTGAA CTCTTGGGAT  360
GATCCTCTAG AGTCCTGTGA TAAATGTCAG CTAAAAGGAG AAGCCATGCA TGGACAAGTA  420
GATTGTAGTC CAGGAATATG GCAACTAGAT TGCACACATT TAGAAGGAAA AATTATCCTG  480
GTAGCAGTTC ATGTAGCCAG TGGCTATATA GAAGCAGAAG TTATTCCAGC AGAGACAGGG  540
CAGGAAACAG CATACTTTAT CTTAAAATTA GCAGGAAGAT GGCCAGTAAA AGTAATACAT  600
ACAGACAATG GCAGTAATTT CACCAGTACT ACGGTTAAGG CCGCCTGTTG GTGGGCGGGG  660
ATCAAGCAGG AATTTGGCAT TCCCTACAAT CCCCAAAGTC AAGGAGTAGT AGAATCTATG  720
AATAAAGAAT TAAAGAAAAT TATAGGACAG GTAAGAGATC AGGCTGAACA TCTTAAGACA  780
GCAGTACAAA TGGCAGTATT TATCCACAAT TTTAAAAGAA AAGGGGGGAT TGGGGGGTAC  840
AGTGCAGGGG AAAGAATAGT AGACATAATA GCAACAGACA TACAAACTAA AGAATTACAA  900
AAACAAATTA TAAAAATTCA AAATTTTCGG GTTTATTACA GGGACAGCAG AGATCCACTT  960
TGGAAAGGAC CAGCAAAGCT CCTCTGGAAA GGTGAAGGGG CAGTAGTAAT ACAAGACAAT 1020
AGTGAAATAA AAGTAGTGCC AAGGAGAAAA GCAAAGATCA TTAGGGATTA TGGAAAACAA 1080
TCAGATCGAT CCTCTAGAGT CCAGACCAGA CAATTATTGT CTGGAATAGT GCAACAGCAG 1140
AACAATTTGC TGAGGGCTAT TGAGACGCAA CAACATCTGT TGCAACTCAC GGTCTGGGGC 1200
ATCAAACAGC TCCAGGCAAG AGTCCTGGCT GTGGAAAGAT ACCTACAGGA TCAACGGCTC 1260
CTAGGGATTT GGGGTTGCTC TGGAAAACTC ATCTGCACCA CTACTGTGCC TTGGAACACT 1320
AGTTGGAGTA ATAAATCTCT AGATACAATT TGGCATAACA TGACCTGGAT GGAGTGGGAA 1380
AGAGAAATTG ACAATTACAC AAGCTCTGAC AAAGGAAACA GCAGCCAGGT CAGTCAAAAT 1440
TACCCTATAG TGCAGAACCT ACAGGGGCAA ATGGTACATC AGGCCATATC ACCTAGAACT 1500
TTAAATGCAT GGGTAAAAGT AATAGAAGAA AAGGCTTTCA GCCCAGAAGT GATACCCATG 1560
TTTTCAGCAT TATCAGAAGG AGCCACCCCA CAAGATTTAA ATACCATGCT AAACACAGTG 1620
GGGGGACATC AAGCAGCCAT GCAAATGTTA AAAGAGACCA TCAATGAGGA AGCTGCAGAA 1680
TGGGATAGAG TGCATCCAGT GCATGCAGGG CCTATTGCAC CAGGCCAGAT GAGAGAACCA 1740
```

AGGGGAAGTG ATATAGCAGG AACTACTAGT ACCCTTCAGG AACAAATAGG ATGGATGACA 1800
AACAATCCAC CTATCCCAGT AGGAGAAATC TATAAAAGAT GGATAATCCT GGGATTAAAT 1860
AAAATAGTAA GAATGTATAG TCCTGTTAGT ATTCTGGACA TAAGACAAGG ACCAAAGGAA 1920
CCCTTTAGAG ACTATGTAGA TCGGTTCTAT AAAACTTTAA GAGCCGAGCA AGCTTCACAG 1980
GAGGTAAAAA ATTGGATGAC AGAAACCTTG TTGGTCCAAA ATGCGAACCC AGACTGTAAG 2040
ACTATTCTAA AAGCATTAGG ACCAGCAGCT ACACTAGAAG AAATGATGAC AGCATGTCAG 2100
GGAGTGGGGG GACCCGGCCA TAAGGCAAGA GTGTTGGCTG AAGCAATGAG CCAAGTAACA 2160
AATTCAGCTA CCATAATGAT GCAGAGAGGT AATTTTAGGA ACCAAAAAAA AACTGTTAAG 2220
TGTTTCAATT GTGGCAAAGA AGGGCACATA GCCAAAAATT GCAGGGCCTC TCGTAAAAAG 2280
CGTAGACGTA AAAAACGTCG TAAAAAGAAA TAG 2313

SEQ ID NO:17 (Oligonukleotid 1)
ART DER SEQUENZ: Nukleotidsequenz
SEQUENZLÄNGE: 100 Basen
STRANGFORM: Einzelstrang
TOPOLOGIE: Linear

BamHI
CGCGGGATCC AGAATTCCAA CAGCAACAGC AGTTGTTGGA CGTTGTTAAA
CGTCAACAGG AACTGTTGCG TCTGACCGTT TGGGGAACCA AGAACCTTCA

SEQ ID NO:18 (Oligonukleotid 2)
ART DER SEQUENZ: Nukleotidsequenz
SEQUENZLÄNGE: 100 Basen
STRANGFORM: Einzelstrang
TOPOLOGIE: Linear

GGCTAGAGTT ACCGCTATCG AAAAATACCT TCAAGACCAG GCTCGTTTGA
KpnI
ACTCCTGGGG TTGCGCTTTT AGACAGGTTT GTCATACCAC GGTACCCGCG

SEQ ID NO:19 (Oligonukleotid 3)
ART DER SEQUENZ: Nukleotidsequenz
SEQUENZLÄNGE: 96 Basen
STRANGFORM: Einzelstrang
TOPOLOGIE: Linear

```
    KpnI
CGCGGGTACC GTGGGTTAAC GACTCTCTGG CTCCAGACTG GGACAACATG

ACCTGGCAGG AATGGGAAAA GCAAGTTCGT TACTTGGAAG CTAACA
```

SEQ ID NO:20 (Oligonukleotid 4)
ART DER SEQUENZ: Nukleotidsequenz
SEQUENZLÄNGE: 97 Basen
STRANGFORM: Einzelstrang
TOPOLOGIE: Linear

```
TCTCCAAATC TCTGGAACAG GCTCAAATCC AGCAAGAAAA AAACATGTAC
                                           HindIII
GAACTGCAGA AGTTGAACTC TTGGGATATC AGATCTAAGC TTTGGGC
```

SEQ ID NO:21 (Oligonukleotid 5)
ART DER SEQUENZ: Nukleotidsequenz
SEQUENZLÄNGE: 48 Basen
STRANGFORM: Einzelstrang
TOPOLOGIE: Linear

```
    HindIII
GCCCAAGCTT AGATCTGATA TCCCAAGAGT TCAACTTCTG CAGTTCGT
```

SEQ ID NO:22 (Oligonukleotid 6)
ART DER SEQUENZ: Nukleotidsequenz
SEQUENZLÄNGE: 96 Basen
STRANGFORM: Einzelstrang
TOPOLOGIE: Linear

ACATGTTTTT TTCTTGCTGG ATTTGAGCCT GTTCCAGAGA TTTGGAGATG
TTAGCTTCCA AGTAACGAAC TTGCTTTTCC CATTCCTGCC AGGTCA

SEQ ID NO:23 (Oligonukleotid 7)
ART DER SEQUENZ: Nukleotidsequenz
SEQUENZLÄNGE: 48 Basen
STRANGFORM: Einzelstrang
TOPOLOGIE: Linear

KpnI
TGTTGTCCCA GTCTGGAGCC AGAGAGTCGT TAACCCACGG TACCCGCG

SEQ ID NO:24 (Oligonukleotid 8)
ART DER SEQUENZ: Nukleotidsequenz
SEQUENZLÄNGE: 50 Basen
STRANGFORM: Einzelstrang
TOPOLOGIE: Linear

KpnI
CGCG GGTACC GTGGTATGAC AAACCTGTCT AAAAGCGCAA CCCCAGGAGT

SEQ ID NO:25 (Oligonukleotid 9)
ART DER SEQUENZ: Nukleotidsequenz
SEQUENZLÄNGE: 100 Basen
STRANGFORM: Einzelstrang
TOPOLOGIE: Linear

TCAAACGAGC CTGGTCTTGA AGGTATTTTT CGATAGCGGT AACTCTAGCC

TGAAGGTTCT TGGTTCCCCA AACGGTCAGA CGCAACAGTT CCTGTTGACG

SEQ ID NO:26 (Oligonukleotid 10)
ART DER SEQUENZ: Nukleotidsequenz
SEQUENZLÄNGE: 50 Basen
STRANGFORM: Einzelstrang
TOPOLOGIE: Linear

BamHI
TTTAACAACG TCCAACAACT GCTGTTGCTG TTGGAATTCT GGATCCCGCG

**Patentansprüche**

**Patentansprüche für folgende Vertragsstaaten : AT, BE, CH, LI, DE, DK, FR, GB, GR, IT, LU, NL, SE**

1. Fusionsprotein mit mindestens einer antigenen oder/und immunogenen Determinante aus der env-, gag- oder/und pol-Region von HIV1 oder/und HIV2,
**dadurch gekennzeichnet**,
daß es als N-terminalen Bestandteil die Tetrapeptid-Sequenz $NH_2$-Met-Tyr-Tyr-Leu enthält.

2. Fusionsprotein nach Anspruch 1,
**dadurch gekennzeichnet**,
daß es die Aminosäuresequenz I (SEQ ID NO: 1) mit einer Determinante aus der gag-Region von HIV1 besitzt.

3. Fusionsprotein nach Anspruch 1,
**dadurch gekennzeichnet**,
daß es die Aminosäuresequenz II (SEQ ID NO: 2) mit einer Determinante aus der pol-Region von HIV1 besitzt.

4. Fusionsprotein nach Anspruch 1,
**dadurch gekennzeichnet**,
daß es die Aminosäuresequenz III (SEQ ID NO: 3) mit einer Determinante aus der env-Region von HIV1 besitzt.

5. Fusionsprotein nach Anspruch 1,
**dadurch gekennzeichnet**,
daß es die Aminosäuresequenz IV (SEQ ID NO: 4) mit einer Determinante aus der env-Region von HIV2 besitzt.

**6.** Fusionsprotein nach Anspruch 1,
**dadurch gekennzeichnet**,
daß es die Aminosäuresequenz V (SEQ ID NO: 5) mit Determinanten aus der env- und der gag-Region von HIV1 besitzt.

**7.** Fusionsprotein nach Anspruch 1,
**dadurch gekennzeichnet**,
daß es die Aminosäuresequenz VI (SEQ ID NO: 6) mit Determinanten aus der env-Region von HIV2 und der pol-Region von HIV1 besitzt.

**8.** Fusionsprotein nach Anspruch 1,
**dadurch gekennzeichnet**,
daß es die Aminosäuresequenz VII (SEQ ID NO: 7) mit Determinanten aus der env-Region von HIV2, der pol-, env- und gag-Region von HIV1 besitzt.

**9.** Fusionsprotein nach Anspruch 1,
**dadurch gekennzeichnet**,
daß es die Aminosäuresequenz VIII (SEQ ID NO: 8) mit Determinanten aus der env-Region von HIV2, der pol-, env- und gag-Region von HIV1 und C-terminal eine poly (Lys, Arg) Sequenz enthält.

**10.** Rekombinante DNA,
**dadurch gekennzeichnet**,
daß sie für ein Fusionsprotein nach einem der Ansprüche 1 bis 9 kodiert.

**11.** Rekombinanter Vektor,
**dadurch gekennzeichnet**,
daß er mindestens eine Kopie einer oder mehrerer rekombinanter DNA-Sequenzen nach Anspruch 10 enthält.

**12.** Mikroorganismus,
**dadurch gekennzeichnet**,
daß er mit einer rekombinanten DNA nach Anspruch 10 oder einem rekombinanten Vektor nach Anspruch 11 transformiert ist.

**13.** Verfahren zur Gewinnung eines Fusionsproteins nach einem der Ansprüche 1 bis 9,
**dadurch gekennzeichnet**,
daß man Mikroorganismen, vorzugsweise E. coli-Zellen mit einer rekombinanten DNA nach Anspruch 10 oder einem rekombinanten Vektor nach Anspruch 11 transformiert und ein Fusionsprotein nach Anspruch 1 nach üblichen biochemischen Verfahren aus den Zellen oder dem Medium gewinnt.

**14.** HIV-Antikörper-Nachweisreagenz, enthaltend:
(a) eines oder mehrere Fusionsproteine mit mindestens einer antigenen oder/und immunogenen Determinante von HIV1 oder/und HIV2 nach einem der Ansprüche 1 bis 9, die entweder an eine Festphase gebunden sind oder so modifiziert sind, daß sie während eines Inkubationsschritts leicht an eine geeignete spezielle Festphase binden,
(b) einen Inkubationspuffer, der die physiologische Bindung von Anti-HIV-Antikörpern an die antigenen Determinanten der Fusionsproteine erlaubt und gleichzeitig unspezifische Bindungen unterdrückt,
(c) ein Nachweisreagenz zur Erkennung der Antigen-gebundenen Antikörper und
(d) ein System zur Quantifizierung der gebundenen spezifischen Antikörper.

**Patentansprüche für folgenden Vertragsstaat : ES**

**1.** Verfahren zur Gewinnung eines Fusionsproteins mit mindestens einer antigenen oder/und immunogenen Determinante aus der env-, gag- oder/und pol-Region von HIV1 oder/und HIV2, das als N-terminalen Bestandteil die Tetrapeptid-Sequenz $NH_2$-Met-Tyr-Tyr-Leu enthält,

**dadurch gekennzeichnet,**
daß man Mikroorganismen, vorzugsweise E.coli-Zellen mit einer rekombinanten DNA, die für das Fusionsprotein kodiert, oder einem rekombinanten Vektor, der mindestens eine Kopie einer oder mehrerer für das Fusionsprotein kodierender rekombinanter DNA-Sequenzen enthält, transformiert und das Fusionsprotein nach üblichen biochemischen Verfahren aus den Zellen oder dem Medium gewinnt.

**2.** Verfahren nach Anspruch 1,
**dadurch gekennzeichnet,**
daß das Fusionsprotein die Aminosäuresequenz I (SEQ ID NO: 1) mit einer Determinante aus der gag-Region von HIV1 besitzt.

**3.** Verfahren nach Anspruch 1,
**dadurch gekennzeichnet,**
daß das Fusionsprotein die Aminosäuresequenz II (SEQ ID NO: 2) mit einer Determinante aus der pol-Region von HIV1 besitzt.

**4.** Verfahren nach Anspruch 1,
**dadurch gekennzeichnet,**
daß das Fusionsprotein die Aminosäuresequenz III (SEQ ID NO: 3) mit einer Determinante aus der env-Region von HIV1 besitzt.

**5.** Verfahren nach Anspruch 1,
**dadurch gekennzeichnet,**
daß das Fusionsprotein die Aminosäuresequenz IV (SEQ ID NO: 4) mit einer Determinante aus der env-Region von HIV2 besitzt.

**6.** Verfahren nach Anspruch 1,
**dadurch gekennzeichnet,**
daß das Fusionsprotein die Aminosäuresequenz V (SEQ ID NO: 5) mit Determinanten aus der env- und der gag-Region von HIV1 besitzt.

**7.** Verfahren nach Anspruch 1,
**dadurch gekennzeichnet,**
daß das Fusionsprotein die Aminosäuresequenz VI (SEQ ID NO: 6) mit Determinanten aus der env-Region von HIV2 und der pol-Region von HIV1 besitzt.

**8.** Verfahren nach Anspruch 1,
**dadurch gekennzeichnet,**
daß das Fusionsprotein die Aminosäuresequenz VII (SEQ ID NO: 7) mit Determinanten aus der env-Region von HIV2, der pol-, env- und gag-Region von HIV1 besitzt.

**9.** Verfahren nach Anspruch 1,
**dadurch gekennzeichnet,**
daß das Fusionsprotein die Aminosäuresequenz VIII (SEQ ID NO: 8) mit Determinanten aus der env-Region von HIV2, der pol-, env- und gag-Region von HIV1 und C-terminal eine poly(Lys, Arg) Sequenz enthält.

**10.** HIV-Antikörper-Nachweisreagenz, enthaltend:
(a) eines oder mehrere, durch ein Verfahren nach einem der Ansprüche 1 bis 9 gewonnene Fusionsproteine mit mindestens einer antigenen oder/und immunogenen Determinante von HIV1 oder/und HIV2, die entweder an eine Festphase gebunden sind oder so modifiziert sind, daß sie während eines Inkubationsschritts leicht an eine geeignete spezielle Festphase binden,
(b) einen Inkubationspuffer, der die physiologische Bindung von Anti-HIV-Antikörpern an die antigenen Determinanten der Fusionsproteine erlaubt und gleichzeitig unspezifische Bindungen unterdrückt,
(c) ein Nachweisreagenz zur Erkennung der Antigen-gebundenen Antikörper und
(d) ein System zur Quantifizierung der gebundenen spezifischen Antikörper.

## Claims

**Claims for the following Contracting States : AT, BE, CH, LI, DE, DK, FR, GB, GR, IT, LU, NL, SE**

1. Fusion protein with at least one antigenic or/and immunogenic determinant from the env, gag or/and pol region of HIV1 or/and HIV2,
**wherein**
it contains the tetrapeptide sequence $NH_2$-Met-Tyr-Tyr-Leu as the N-terminal component.

2. Fusion protein as claimed in claim 1,
**wherein**
it has the amino acid sequence I (SEQ ID NO:1) with one determinant from the gag region of HIV1.

3. Fusion protein as claimed in claim 1,
**wherein**
it has the amino acid sequence II (SEQ ID NO:2) with one determinant from the pol region of HIV1.

4. Fusion protein as claimed in claim 1,
**wherein**
it has the amino acid sequence III (SEQ ID NO:3) with one determinant from the env region of HIV1.

5. Fusion protein as claimed in claim 1,
**wherein**
it has the amino acid sequence IV (SEQ ID NO:4) with one determinant from the env region of HIV2.

6. Fusion protein as claimed in claim 1,
**wherein**
it has the amino acid sequence V (SEQ ID NO:5) with determinants from the env and the gag region of HIV1.

7. Fusion protein as claimed in claim 1,
**wherein**
it has the amino acid sequence VI (SEQ ID NO:6) with determinants from the env region of HIV2 and the pol region of HIV1.

8. Fusion protein as claimed in claim 1,
**wherein**
it has the amino acid sequence VII (SEQ ID NO:7) with determinants from the env region of HIV2 and from the pol, env, and gag region of HIV1.

9. Fusion protein as claimed in claim 1,
**wherein**
it has the amino acid sequence VIII (SEQ ID NO:8) with determinants from the env region of HIV2 and from the pol, env and gag region of HIV1 and contains a C-terminal poly (Lys, Arg) sequence.

10. Recombinant DNA,
**wherein**
it codes for a fusion protein as claimed in one of the claims 1 to 9.

11. Recombinant vector,
**wherein**
it contains at least one copy of one or several recombinant DNA sequences as claimed in claim 10.

12. Microorganism,
**wherein**
it is transformed with a recombinant DNA as claimed in claim 10 or with a recombinant vector as claimed in claim 11.

**13.** Process for the production of a fusion protein as claimed in one of the claims 1 to 9,
**wherein**
microorganisms, preferably E. coli cells, are transformed with a recombinant DNA as claimed in claim 10 or a recombinant vector as claimed in claim 11 and a fusion protein as claimed in claim 1 is isolated from the cells or the medium according to conventional biochemical procedures.

**14.** HIV antibody test reagent containing:
(a) one or several fusion proteins with at least one antigenic or/and immunogenic determinant of HIV1 or/and HIV2 as claimed in one of the claims 1 to 9, which are either bound to a solid phase or are modified in such a manner that they easily bind to a suitable special solid phase during an incubation step,
(b) an incubation buffer which allows the physiological binding of anti-HIV antibodies to the antigenic determinants of the fusion proteins and which at the same time suppresses non-specific binding,
(c) a test reagent for the recognition of antigen-bound antibodies and
(d) a system for the quantification of the bound specific antibodies.

**Claims for the following Contracting State : ES**

**1.** Process for producing a fusion protein with at least one antigenic or/and immunogenic determinant from the env, gag or/and pol region of HIV1 or/and HIV2 which contains the tetrapeptide sequence $NH_2$-Met-Tyr-Tyr-Leu as the N-terminal component,
**wherein**
microorganisms, preferably E. coli cells, are transformed with a recombinant DNA which codes for the fusion protein or with a recombinant vector which contains at least one copy of one or several recombinant DNA sequences coding for the fusion protein and a fusion protein is isolated from the cells or the medium according to conventional biochemical procedures.

**2.** Process as claimed in claim 1,
**wherein**
the fusion protein has the amino acid sequence I (SEQ ID NO:1) with one determinant from the gag region of HIV1.

**3.** Process as claimed in claim 1,
**wherein**
the fusion protein has the amino acid sequence II (SEQ ID NO:2) with one determinant from the pol region of HIV1.

**4.** Process as claimed in claim 1,
**wherein**
the fusion protein has the amino acid sequence III (SEQ ID NO:3) with one determinant from the env region of HIV1.

**5.** Process as claimed in claim 1,
**wherein**
the fusion protein has the amino acid sequence IV (SEQ ID NO:4) with one determinant from the env region of HIV2.

**6.** Process as claimed in claim 1,
**wherein**
the fusion protein has the amino acid sequence V (SEQ ID NO:5) with determinants from the env and the gag region of HIV1.

**7.** Process as claimed in claim 1,
**wherein**
the fusion protein has the amino acid sequence VI (SEQ ID NO:6) with determinants from the env region of HIV2 and the pol region of HIV1.

**8.** Process as claimed in claim 1, **wherein** the fusion protein has the amino acid sequence VII (SEQ ID NO:7) with determinants from the env region of HIV2 and from the pol, env, and gag region of HIV1.

**9.** Process as claimed in claim 1, **wherein** the fusion protein has the amino acid sequence VIII (SEQ ID NO:8) with determinants from the env region of HIV2 and from the pol, env and gag region of HIV1 and contains a C-terminal poly (Lys, Arg) sequence.

**10.** HIV antibody test reagent containing:

(a) one or several fusion proteins obtained by a process as claimed in one of the claims 1 to 9 with at least one antigenic or/and immunogenic determinant of HIV1 or/and HIV2, which are either bound to a solid phase or are modified in such a manner that they easily bind to a suitable special solid phase during an incubation step,
(b) an incubation buffer which allows the physiological binding of anti-HIV antibodies to the antigenic determinants of the fusion proteins and which at the same time suppresses non-specific binding,
(c) a test reagent for the recognition of antigen-bound antibodies and
(d) a system for the quantification of the bound specific antibodies.

**Revendications**

**Revendications pour les Etats contractants suivants : AT, BE, CH, LI, DE, DK, FR, GB, GR, IT, LU, NL, SE**

**1.** Protéine de fusion comportant au moins un déterminant antigénique et/ou immunogène provenant de la région env, gag et/ou pol de VIH1 et/ou de VIH2, caractérisée en ce qu'elle contient comme constituant N-terminal la séquence tétrapeptidique $NH_2$-Met-Tyr-Tyr-Leu.

**2.** Protéine de fusion selon la revendication 1, caractérisée en ce qu'elle possède la séquence d'acides aminés I (SEQ ID NO: 1) comportant un déterminant provenant de la région gag de VIH1.

**3.** Protéine de fusion selon la revendication 1, caractérisée en ce qu'elle possède la séquence d'acides aminés II (SEQ ID NO: 2) comportant un déterminant provenant de la région pol de VIH1.

**4.** Protéine de fusion selon la revendication 1, caractérisée en ce qu'elle possède la séquence d'acides aminés III (SEQ ID NO: 3) comportant un déterminant provenant de la région env de VIH1.

**5.** Protéine de fusion selon la revendication 1, caractérisée en ce qu'elle possède la séquence d'acides aminés IV (SEQ ID NO: 4) comportant un déterminant provenant de la région env de VIH2.

**6.** Protéine de fusion selon la revendication 1, caractérisée en ce qu'elle possède la séquence d'acides aminés V (SEQ ID NO: 5) comportant des déterminants provenant de la région env et de la région gag de VIH1.

**7.** Protéine de fusion selon la revendication 1, caractérisée en ce qu'elle possède la séquence d'acides aminés VI (SEQ ID NO: 6) comportant des déterminants provenant de la région env de VIH2 et de la région pol de VIH1.

**8.** Protéine de fusion selon la revendication 1, caractérisée en ce qu'elle possède la séquence d'acides aminés VII (SEQ ID NO: 7) comportant des déterminants provenant de la région env de VIH2 et de la région pol, env et gag de VIH1.

**9.** Protéine de fusion selon la revendication 1, caractérisée en ce qu'elle possède la séquence d'acides aminés VIII (SEQ ID NO: 8) comportant des déterminants provenant de la région env de VIH2, de la région pol, env et gag de VIH1 et à l'extrémité C-terminale une séquence poly(Lys, Arg).

**10.** ADN recombiné, caractérisé en ce qu'il code une protéine de fusion selon l'une des revendications 1 à 9.

**11.** Vecteur recombiné, caractérisé en ce qu'il contient au moins une copie d'une ou plusieurs séquences d'ADN recombiné selon la revendication 10.

**12.** Microorganisme caractérisé en ce qu'il est transformé avec un ADN recombiné selon la revendication 10 ou avec un vecteur recombiné selon la revendication 11.

**13.** Procédé d'obtention d'une protéine de fusion selon l'une des revendications 1 à 9, caractérisé en ce que l'on transforme des micro-organismes, de préférence des cellules de E. coli, avec un ADN recombiné selon la revendication 10 ou avec un vecteur recombiné selon la revendication 11 et on obtient à partir des cellules ou du milieu une protéine de fusion selon la revendication 1 selon des procédés biochimiques habituels.

**14.** Réactif de mise en évidence d'anticorps anti-VIH, contenant:
(a) une ou plusieurs protéines de fusion comportant au moins un déterminant antigénique et/ou immunogène de VIH1 et/ou de VIH2 selon l'une des revendications 1 à 9 , qui sont liées à une phase solide ou qui sont modifiées de telle manière que, pendant une étape d'incubation, elles se lient aisément à une phase solide spéciale appropriée,
(b) un tampon d'incubation qui permet la liaison physiologique d'anticorps anti-VIH aux déterminants antigéniques des protéines de fusion et qui empêche en même temps des liaisons non spécifiques,
(c) un réactif de mise en évidence pour déceler les anticorps liés aux antigènes et
(d) un système pour quantifier les anticorps spécifiques liés.

**Revendications pour l'Etat contractant suivant : ES**

**1.** Procédé d'obtention d'une protéine de fusion comportant au moins un déterminant antigénique et/ou immunogène provenant de la région env, gag et/ou pol de VIH1 et/ou de VIH2, qui contient comme constituant N-terminal la séquence tétrapeptidique $NH_2$-Met-Tyr-Tyr-Leu, caractérisé en ce que l'on transforme des microorganismes, de préférence des cellules de E. coli, avec un ADN recombiné qui code la protéine de fusion ou avec un vecteur recombiné qui contient au moins une copie d'une ou plusieurs séquences d'ADN recombiné codant la protéine de fusion et on obtient à partir des cellules ou du milieu la protéine de fusion selon des procédés biochimiques habituels.

**2.** Procédé selon la revendication 1, caractérisé en ce que la protéine de fusion possède la séquence d'acides aminés I (SEQ ID NO: 1) comportant un déterminant provenant de la région gag de VIH1.

**3.** Procédé selon la revendication 1, caractérisé en ce que la protéine de fusion possède la séquence d'acides aminés II (SEQ ID NO: 2) comportant un déterminant provenant de la région pol de VIH1.

**4.** Procédé selon la revendication 1, caractérisé en ce que la protéine de fusion possède la séquence d'acides aminés III (SEQ ID NO: 3) comportant un déterminant provenant de la région env de VIH1.

**5.** Procédé selon la revendication 1, caractérisé en ce que la protéine de fusion possède la séquence d'acides aminés IV (SEQ ID NO: 4) comportant un déterminant provenant de la région env de VIH2.

**6.** Procédé selon la revendication 1, caractérisé en ce que la protéine de fusion possède la séquence d'acides aminés V (SEQ ID NO: 5) comportant des déterminants provenant de la région env et de la région gag de VIH1.

**7.** Procédé selon la revendication 1, caractérisé en ce que la protéine de fusion possède la séquence d'acides aminés VI (SEQ ID NO: 6) comportant des déterminants provenant de la région env de VIH2 et de la région pol de VIH1.

**8.** Procédé selon la revendication 1, caractérisé en ce que la protéine de fusion possède la séquence d'acides aminés VII (SEQ ID NO: 7) comportant des déterminants provenant de la région env de VIH2 et de la région pol, env et gag de VIH1.

**9.** Procédé selon la revendication 1, caractérisé en ce que la protéine de fusion possède la séquence d'acides aminés VIII (SEQ ID NO: 8) comportant des déterminants provenant de la région env de VIH2, de la région pol, env et gag de VIH1 et à l'extrémité C-terminale une séquence poly(Lys, Arg).

**10.** Réactif de mise en évidence d'anticorps anti-VIH, contenant:

(a) une ou plusieurs protéines de fusion obtenues par un procédé selon l'une des revendications 1 à 9 comportant au moins un déterminant antigénique et/ou immunogène de VIH1 et/ou de VIH2, qui sont liées à une phase solide ou qui sont modifiées de telle manière que, pendant une étape d'incubation, elles se lient aisément à une phase solide spéciale appropriée,

(b) un tampon d'incubation qui permet la liaison physiologique d'anticorps anti-VIH aux déterminants antigéniques des protéines de fusion et qui empêche en même temps des liaisons non spécifiques,

(c) un réactif de mise en évidence pour déceler les anticorps liés aux antigènes et

(d) un système pour quantifier les anticorps spécifiques liés.

Fig.1

Lokalisierung der HIV-Determinanten
auf dem HIV1-Genom

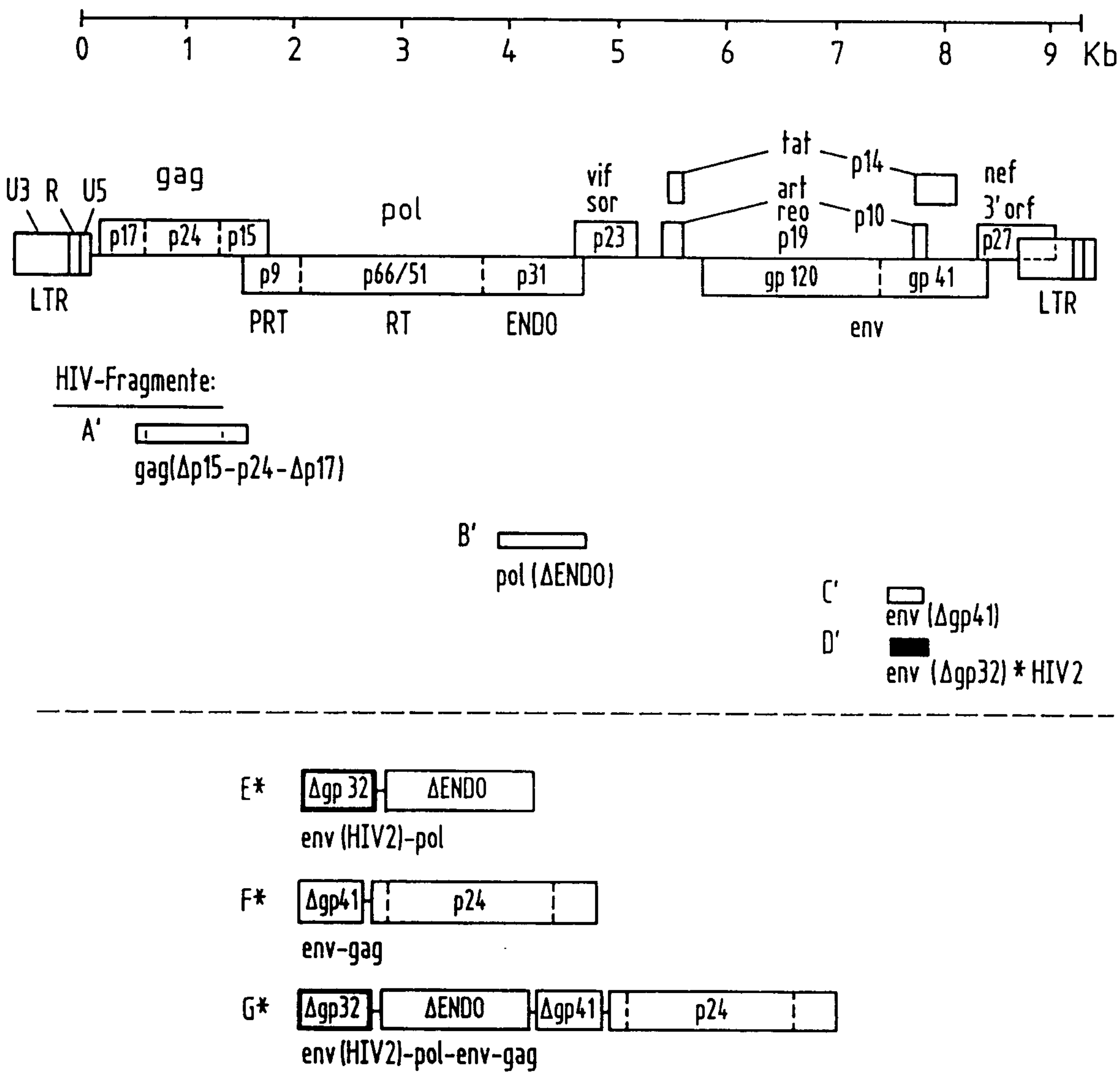

# Fig. 2

Klonierungsstrategie

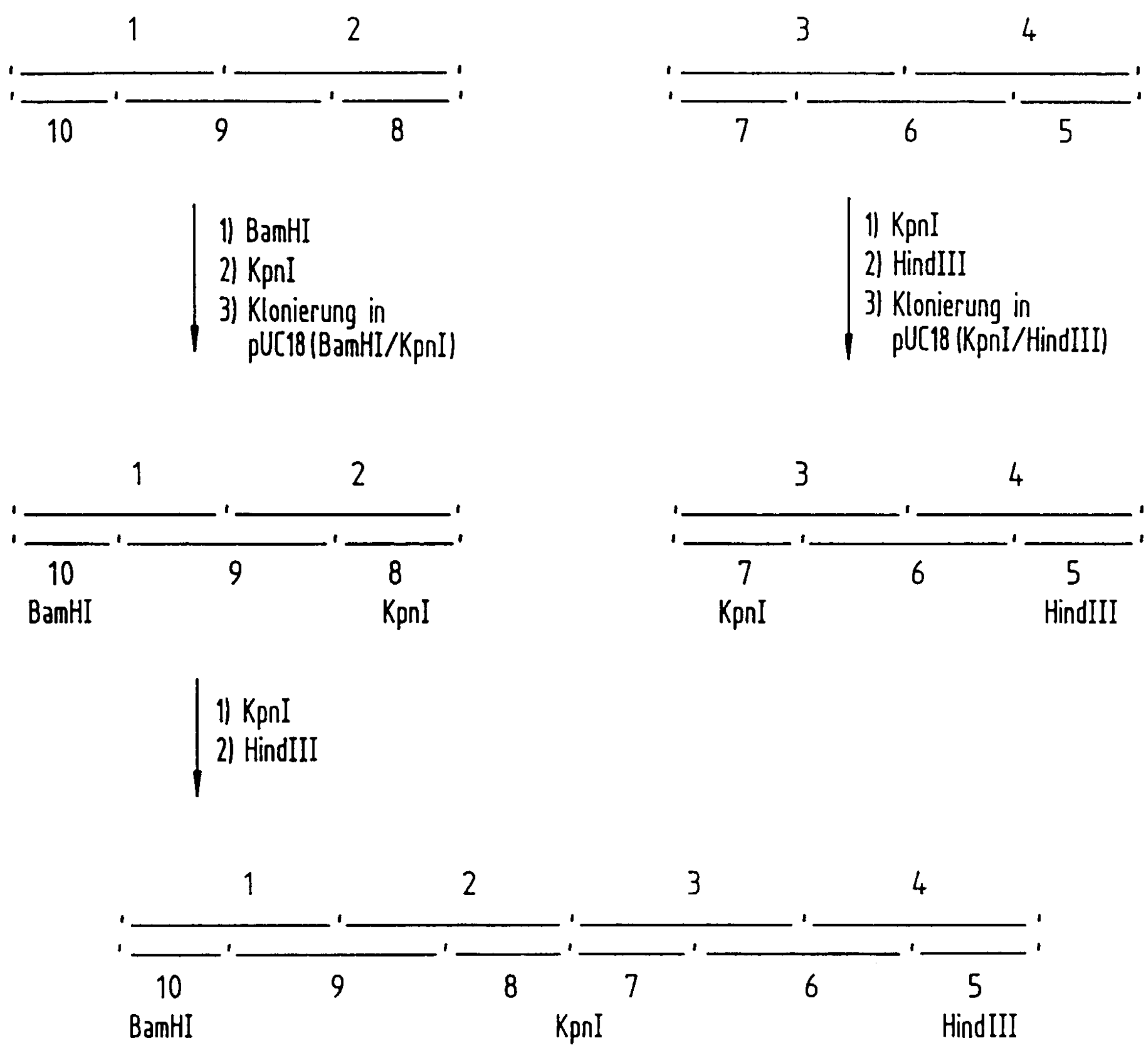


DNA-Fragment D'